(19) Europäisches Patentamt
European Patent Office
Office européen des brevets

(11) Veröffentlichungsnummer: **0 114 347**
**A2**

(12) # EUROPÄISCHE PATENTANMELDUNG

(21) Anmeldenummer: 83112847.5

(22) Anmeldetag: 20.12.83

(51) Int. Cl.³: C 07 D 249/04
A 61 K 31/41
//C07C117/00

(30) Priorität: 23.12.82 CH 7526/82

(43) Veröffentlichungstag der Anmeldung: 01.08.84 Patentblatt 84/31

(84) Benannte Vertragsstaaten: AT BE CH DE FR GB IT LI LU NL SE

(71) Anmelder: CIBA-GEIGY AG
Postfach
CH-4002 Basel(CH)

(72) Erfinder: Meier, René
Rickenbacherstrasse 76
CH-4463 Buus(CH)

(74) Vertreter: Zumstein, Fritz sen., Dr. et al,
Bräuhausstrasse 4
D-8000 München 2(DE)

(54) **Aralkyltriazolverbindungen.**

(57) 1-Phenylniederalkyl-1H-1,2,3-triazolverbindungen der Formel

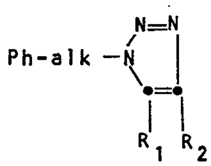

(I),

worin Ph durch Niederalkyl, Halogen und/oder Trifluormethyl substituiertes Phenyl bedeutet, alk Niederalkyliden darstellt, $R_1$ Wasserstoff, Niederalkyl, Niederalkoxy oder eine unsubstituierte oder durch Acyl, Niederalkyl oder Niederalkylen bzw. Aza-, Oxa- oder Thianiederalkylen substituierte Amino- oder Carbamylgruppe bedeutet und $R_2$ für eine unsubstituierte oder durch Acyl, Niederalkyl, Niederalkylen bzw. Aza-, Oxa- oder Thianiederalkylen substituierte Carbamylgruppe oder sofern $R_1$ eine unsubstituierte oder durch Acyl, Niederalkyl oder Niederalkylen bzw. Aza-, Oxa- oder Thianiederalkylen substituierte Aminogruppe darstellt, für Cyano bzw., sofern $R_1$ eine unsubstituierte oder durch Acyl, Niederalkyl oder Niederalkylen bzw. Aza-, Oxa- oder Thianiederalkylen substituierte Carbamylgruppe bedeutet, für Wasserstoff oder Niederalkyl steht haben antikonvulsive Eigenschaften und sind als Arzneimittel verwendbar.

EP 0 114 347 A2

CIBA-GEIGY AG
Basel (Schweiz)

4-14247/+

## Aralkyltriazolverbindungen

Die Erfindung betrifft neue Aralkyltriazolverbindungen, insbesondere 1-Phenylniederalkyl-1H-1,2,3-triazolverbindungen der Formel

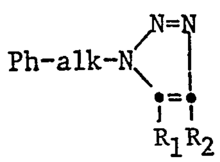

(I),

worin Ph durch Niederalkyl, Halogen und/oder Trifluormethyl substituiertes Phenyl bedeutet, alk Niederalkyliden darstellt, $R_1$ Wasserstoff, Niederalkyl, Niederalkoxy oder eine unsubstituierte oder durch Acyl, Niederalkyl oder Niederalkylen bzw. Aza-, Oxa- oder Thianiederalkylen substituierte Amino- oder Carbamylgruppe bedeutet und $R_2$ für eine unsubstituierte oder durch Acyl, Niederalkyl oder Niederalkylen bzw. Aza-, Oxa- oder Thianiederalkylen substituierte Carbamylgruppe oder, sofern $R_1$ eine unsubstituierte oder durch Acyl, Niederalkyl oder Niederalkylen bzw. Aza-, Oxa- oder Thianiederalkylen substituierte Aminogruppe darstellt, für Cyano bzw., sofern $R_1$ eine unsubstituierte oder durch Acyl, Niederalkyl oder Niederalkylen bzw. Aza-, Oxa- oder Thianiederalkylen substituierte Carbamylgruppe bedeutet, für Wasserstoff oder Niederalkyl steht, und Salze von salzbildenden Verbindungen der Formel I sowie Verfahren zur Herstellung der neuen Verbindungen, diese enthaltende pharmazeutische Präparate und ihre Verwendung.

Der Phenylrest Ph enthält beispielsweise bis und mit drei, insbesondere einen oder zwei, der genannten Substituenten.

Die Erfindung betrifft beispielsweise solche Verbindungen der Formel I, worin Ph in 2-Stellung durch Niederalkyl, Halogen oder Trifluormethyl und gegebenenfalls zusätzlich durch Niederalkyl oder Halogen, welches sich beispielsweise in 3-, 4- oder 6-Stellung, vorzugsweise in 3- oder 6-Stellung befindet, substituiertes Phenyl bedeutet, alk Niederalkyliden darstellt, $R_1$ Wasserstoff, Niederalkyl, Niederalkoxy oder eine unsubstituierte oder durch Niederalkyl substituierte Amino- bzw. Carbamylgruppe darstellt und $R_2$ für unsubstituiertes oder durch Niederalkyl substituiertes Carbamyl oder, sofern $R_1$ unsubstituiertes oder durch Niederalkyl substituiertes Amino darstellt, für Cyano steht, Verfahren zu ihrer Herstellung, diese enthaltende pharmazeutische Präparate und ihre Verwendung.

Gegebenenfalls durch Acyl, Niederalkyl, Niederalkylen bzw. Aza-, Oxa- oder Thianiederalkylen substituiertes Amino ist beispielsweise Amino, physiologisch leicht spaltbares Acylamino, Niederalkylamino, Diniederalkylamino, 5- bis 7-gliedriges Niederalkylenamino bzw. Aza-, Oxa- oder Thianiederalkylenamino. Ebenso ist entsprechend substituiertes Carbamyl beispielsweise Carbamyl, physiologisch leicht spaltbares Acylcarbamyl, N-Niederalkylcarbamyl, N,N-Diniederalkylcarbamyl, N,N-Niederalkylen- bzw. N,N-(Aza-, Oxa- oder Thia)-niederalkylencarbamyl mit 4 bis 6 Ringgliedern im Niederalkylen- bzw. Aza-, Oxa- bzw. Thianiederalkylenteil.

In einer physiologisch leicht spaltbaren Acylamino- bzw. Acylcarbamylgruppe leitet sich Acyl insbesondere von einer gegebenenfalls durch Niederalkoxy oder niederalkyliertes Amino substituierten Niederalkansäure, einer Niederalkansulfonsäure oder einem aliphatischen Halbester oder Halbamid der Kohlensäure ab und bedeutet beispielsweise Niederalkanoyl, z.B. Formyl, Acetyl, Propionyl,Butyryl, Isobutyryl, Valeroyl oder Pivaloyl, Niederalkoxyniederalkanoyl, z.B. Methoxy- oder Aethoxyacetyl, Diniederalkylaminoniederalkanoyl, z.B. N,N-Dimethylglycyl, Niederalkansulfonyl, z.B. Methan- oder Aethansulfonyl, Niederalkoxycarbonyl, z.B. Methoxy-, Aethoxy-, Isopropyloxy- oder

Tertiärbutyloxycarbonyl, Ureido, 3-Niederalkyl- bzw. 3,3-Diniederalkylureido oder 3,3-Niederalkylen- bzw. 3,3-(Aza-, Oxa- oder Thia)-niederalkylenureido.

Vor- und nachstehend werden unter "niederen" organischen Gruppen und Verbindungen beispielsweise solche verstanden, die bis und mit 7, insbesondere bis und mit 4, Kohlenstoffatome (C-Atome) aufweisen.

Niederalkyl ist beispielsweise Methyl, Aethyl, Propyl, Isopropyl, Butyl, Isobutyl, Sekundärbutyl oder Tertiärbutyl, ferner eine der isomeren Pentyl-, Hexyl- oder Heptylgruppen.

Halogen ist beispielsweise solches der Atomnummer bis und mit 35, wie Fluor, Chlor oder Brom.

Niederalkyliden ist beispielsweise 1,1-Niederalkyliden mit bis und mit 4 C-Atomen, wie Methylen, Aethyliden, 1,1-Propyliden, 1,1-Butyliden, kann aber auch Isopropyliden, 1,1-Isobutyliden oder eine 1,1-Pentyliden, 1,1-Hexyliden- oder 1,1-Heptylidengruppe sein.

Niederalkoxy ist beispielsweise Methoxy, Aethoxy, Propyloxy, Isopropyloxy, Butyloxy, Isobutyloxy, Sekundärbutyloxy oder Tertiärbutyloxy, ferner eine der isomeren Pentyloxy-, Hexyloxy- oder Heptyloxygruppen.

Niederalkylamino sowie solches in 3-Niederalkylureido ist beispielsweise geradkettiges Niederalkylamino mit bis und mit 4 C-Atomen, wie Methyl-, Aethyl-, Propyl- oder Butylamino, kann aber auch verzweigtes Niederalkylamino mit bis und mit 4 C-Atomen, wie Isopropyl, Isobutyl- oder Tertiärbutylamino, oder eine Pentylamino-, Hexylamino- oder Heptylaminogruppe sein. Diniederalkylamino sowie solches in 3,3-Diniederalkylureido ist beispielsweise geradkettiges Diniederalkylamino

mit jeweils bis und mit 4 C-Atomen, wie Dimethylamino, Diäthylamino, Methyl-äthyl-amino, Dipropylamino oder Dibutylamino, kann aber auch einfach verzweigtes Diniederalkylamino mit jeweils bis und mit 4 C-Atomen, wie Methyl-isopropyl-amino, sein. 5- bis 7-gliedriges Niederalkylenamino bzw. Aza-, Oxa- oder Thianiederalkylenamino, auch als Bestandteil entsprechender Ureidogruppen,ist beispielsweise Pyrrolidino, Piperidino, Piperazino bzw. N'-Niederalkyl-, wie N'-Methylpiperazino, Morpholino oder Thiomorpholino.

N-Niederalkylcarbamyl ist beispielsweise geradkettiges N-Niederalkylcarbamyl mit bis und mit 4 C-Atomen im Niederalkylteil, wie N-Methyl-, N-Aethyl-, N-Propyl- oder N-Butylcarbamyl, kann aber auch verzweigtes N-Niederalkylcarbamyl mit bis und mit 4 Niederalkyl-C-Atomen, wie N-Isopropyl-, N-Isobutyl-, N-Sekundärbutyl- oder N-Tertiärbutylcarbamoyl oder eine Pentylcarbamoyl, Hexylcarbamoyl oder Heptylcarbamoylgruppe sein. N,N-Diniederalkylcarbamyl ist beispielsweise geradkettiges N,N-Diniederalkylcarbamyl mit jeweils bis und mit 4 C-Atomen in den Niederalkylteilen, wie N,N-Dimethyl-, N,N-Diäthyl-, N-Aethyl-N-methyl-, N,N-Dipropyl- oder N,N-Dibutylcarbamyl, kann aber auch einfach verzweigtes N,N-Diniederalkylcarbamyl mit jeweils bis und mit 4 C-Atomen in den Niederalkylteilen, wie N-Isopropyl-N-methyl- oder N-Isobutyl-N-methyl-carbamyl, oder eine N,N-Dipentyl-, N,N-Dihexyl- oder N,N-Diheptylcarbamoylgruppe sein. N,N-Niederalkylen- bzw. N,N-(Aza-, Oxa- oder Thia)-niederalkylencarbamyl ist beispielsweise Pyrrolidinocarbonyl, Piperidinocarbonyl, Piperazino- bzw. N'-Niederalkyl-, wie N'-Methylpiperazinocarbonyl, Morpholinocarbonyl oder Thiomorpholinocarbonyl.

Salzbildende Verbindungen der Formel I sind beispielsweise solche, in denen $R_1$ eine durch Azaniederalkylen substituierte Aminogruppe darstellt und/oder Carbamyl $R_1$ und/oder $R_2$ eine solche aufweist. Als Salze solcher Verbindungen kommen insbesondere deren pharmazeutisch verwendbare Säureadditionssalze mit starken Säuren, wie Mineralsäure, z.B. Salze mit Halogenwasserstoffsäuren, vor allem Chlor-oder Bromwasserstoffsäure, d.h. Hydrohalogenide, vor allem Hydrochloride und Hydrobromide, oder Schwefelsäuresalze, d.h. Hydrogensulfate und Sulfate, ferner Salze mit geeigneten organischen Säuren, wie Dicarbonsäuren oder organischen Sulfonsäuren, z.B. Maleinate, Fumarate, Maleate, Tartrate oder Methansulfonate, ferner N-Cyclohexylsulfaminate, in Betracht.

Die neuen Verbindungen besitzen wertvolle pharmakologische Eigenschaften, insbesondere eine ausgeprägte antikonvulsive Wirksamkeit, die sich beispielsweise an der Maus anhand eines deutlichen Metrazol-Antagonismus im Dosisbereich von etwa 30 bis 300 mg/kg p.o. sowie an Maus und Ratte anhand einer ausgeprägten Schutzwirkung gegen durch Elektroschock ausgelöste Konvulsionen im Dosisbereich von etwa 10-100 mg/kg p.o. (Maus) bzw. von etwa 5-50 mg/kg p.o. (Ratte) zeigen lässt. Die Verbindungen der Formel I sind dementsprechend vorzüglich geeignet zur Behandlung von Konvulsionen verschiedenartiger Genese, beispielsweise zur Behandlung der Epilepsie. Sie können dementsprechend als antikonvulsive, beispielsweise antiepileptische, Arzneimittelwirkstoffe verwendet werden.

Die Erfindung betrifft in erster Linie Verbindungen der Formel I, worin Ph durch Niederalkyl, Halogen und/oder Trifluormethyl substituiertes Phenyl bedeutet, alk Niederalkyliden darstellt, $R_1$ Wasserstoff, Niederalkyl, Niederalkoxy, Amino, Acylamino, N-Niederalkyl-, N,N-Diniederalkyl-, N,N-Niederalkylen- bzw. N,N-(Aza-, Oxa- oder Thia)-niederalkylenamino, Carbamyl, N-Acylcarbamyl oder N-Niederalkyl-, N,N-Diniederalkyl-, N,N-Niederalkylen- bzw. N,N-(Aza-, Oxa- oder Thia)-niederalkylencarbamyl bedeutet und $R_2$ für Carbamyl, N-Acylcarbamyl, N-Niederalkyl-, N,N-Diniederalkyl-, N,N-Niederalkylen- bzw. N,N-(Aza-, Oxa- oder Thia)-niederalkylencarbamyl oder, sofern $R_1$ Amino, Acylamino, N-Niederalkyl-, N,N-Diniederalkyl-, N,N-Niederalkylen- bzw. N,N-(Aza-, Oxa- oder Thia)-niederalkylenamino ist, für Cyano bzw., sofern $R_1$ Carbamyl, N-Acylcarbamyl, N-Niederalkyl-, N,N-Diniederalkyl-, N,N-Niederalkylen- bzw.

N,N-(Aza-, Oxa- oder Thia)-niederalkylencarbamyl bedeutet, für Wasserstoff oder Niederalkyl steht, wobei Acyl jeweils insbesondere Niederalkanoyl, Niederalkoxyniederalkoxycarbonyl, Diniederalkylaminoniederalkanoyl, Niederalkansulfonyl, Niederalkoxycarbonyl, Ureido, 3-Niederalkyl- bzw. 3,3-Diniederalkylureido oder 3,3-Niederalkylen- bzw. 3,3-(Aza-, Oxa- oder Thia)-niederalkylenureido darstellt und Niederalkyl sowie Niederalkyl in N-Niederalkyl- bzw. N,N-Diniederalkylamino bzw. -carbamyl, Niederalkoxy und Niederalkyliden z.B. bis und mit 7 C-Atome und Niederalkylen bzw. Aza-, Oxa- oder Thianiederalkylen z.B. 4 bis und mit 6 Ringglieder aufweisen.

Die Erfindung betrifft in erster Linie z.B. Verbindungen der Formel I, worin Ph 2-Halogenphenyl, 2-Trifluormethylphenyl, 2-Niederalkylphenyl, 2,3- bzw. 2,6-Dihalogenphenyl, 2-Halogen-3-niederalkyl-phenyl bzw. 3-Halogen-2-niederalkylphenyl, 2-Halogen-6-niederalkyl-phenyl, 2,3- bzw. 2,6-Diniederalkylphenyl, 3-Halogen-2-trifluormethyl-phenyl bzw. 2-Halogen-3-trifluormethylphenyl oder 2-Halogen-6-trifluormethylphenyl bedeutet, alk 1,1-Niederalkyliden darstellt, $R_1$ Wasserstoff, Niederalkyl, Niederalkoxy, Amino, Niederalkylamino, Diniederalkylamino, Carbamyl, N-Niederalkylcarbamyl oder N,N-Diniederalkylcarbamyl bedeutet und $R_2$ Carbamyl, N-Niederalkylcarbamyl oder N,N-Diniederalkylcarbamyl bedeutet, wobei Niederalkyl sowie Niederalkyl in N-Niederalkyl bzw. N,N-Diniederalkylamino bzw. -carbamyl, Niederalkoxy und Niederalkyliden z.B. bis und mit 7 C-Atome aufweisen.

Die Erfindung betrifft vor allem Verbindungen der Formel I, worin Ph durch bis und mit 3 Niederalkylgruppen bzw. Halogenatome, Niederalkyl und Halogen und/oder Trifluormethyl, wobei Niederalkyl mit bis und mit 4 C-Atomen aufweist und z.B. Methyl ist, bzw. Halogen die Atomnummer bis und mit 35 aufweist, d.h. Fluor, Chlor oder Brom ist, substituiertes Phenyl bedeutet, das z.B. für 2-Niederalkylphenyl, 2- bzw. 3-Halogenphenyl, 2-Trifluormethylphenyl, 2,3- bzw. 2,6-Diniederalkylphenyl oder 2,3-, 2,4- bzw. 2,6-Dihalogenphenyl steht, alk 1,1-Niederalkyliden mit bis und mit 4 C-Atomen, z.B. Methylen, Aethyliden oder 1,1-Propyliden,

darstellt, $R_1$ Amino, Niederalkanoylamino mit bis und mit 7 C-Atomen, wie Formylamino, Acetylamino oder Pivaloylamino, Niederalkylamino bzw. Diniederalkylamino mit jeweils bis und mit 4 C-Atomen im Alkylteil, z.B. Acetylamino-, Methyl-, Aethyl- oder Propylamino, Dimethyl- oder Diäthylamino, darstellt und $R_2$ Carbamyl, N-Niederalkanoylcarbamyl mit bis und mit 7 C-Atomen, wie Formyl-, Acetyl- oder Pivaloylcarbamyl, N-Niederalkyl-, N,N-Diniederalkyl- oder N,N-Niederalkylencarbamyl mit jeweils bis und mit 4 C-Atomen im Alkyl- bzw. 4 bis und mit 6 Ringgliedern im Alkylenteil, wie Carbamyl, N-Acetylcarbamyl, N-Methylcarbamyl, N,N-Dimethylcarbamyl oder Piperidinocarbonyl, oder Cyano bedeutet.

Die Erfindung betrifft insbesondere Verbindungen der Formel I, worin Ph als Niederalkyl- bzw. Halogensubstituenten Niederalkyl mit bis und mit 4 C-Atomen, z.B. Methyl oder Aethyl, bzw. Halogen der Atomnummer bis und mit 35, z.B. Chlor, aufweisendes 2-Halogenphenyl, wie 2-Fluor-, 2-Chlor- oder 2-Bromphenyl, 2-Trifluormethylphenyl, 2-Niederalkylphenyl, wie 2-Methyl- oder 2-Aethylphenyl, 2,3- oder 2,6-Dihalogenphenyl, z.B. 2,6-Dichlorphenyl, oder 2,3- oder 2,6-Diniederalkylphenyl, wie 2,3-Dimethylphenyl, bedeutet, alk 1,1-Niederalkyliden mit bis und mit 4 C-Atomen, z.B. Methylen, Aethyliden oder 1,1-Propyliden, darstellt, $R_1$ für Amino, Niederalkylamino bzw. Diniederalkylamino mit jeweils bis und mit 4 C-Atomen im Alkylteil, z.B. Methyl-, Aethyl- oder Propylamino, Dimethyl- oder Diäthylamino bedeutet und $R_2$ Carbamyl oder Cyano bedeutet.

Die Erfindung betrifft namentlich die in den Beispielen genannten Verbindungen der Formel I.

Die Verbindungen der Formel I können nach an sich bekannten Methoden hergestellt werden, beispielsweise indem man ein Azid der Formel

$$Ph - alk - N_3 \qquad (II)$$

mit einer Verbindung der Formel

$$Y_1 - \underset{Y_2}{\overset{|}{C}} = \underset{Y_3}{\overset{|}{C}} - R'_2 \qquad \text{(III)},$$

worin $R'_2$ Cyano oder eine unsubstituierte oder durch Acyl, Niederalkyl oder Niederalkylen bzw. Aza-, Oxa- oder Thianiederalkylen substituierte Carbamylgruppe darstellt, $Y_1$ eine wie angegeben disubstituierte Aminogruppe oder, sofern $R'_2$ für eine unsubstituierte oder durch Acyl, Niederalkyl oder Niederalkylen bzw. Aza-, Oxa- oder Thianiederalkylen substituierte Carbamylgruppe steht, Wasserstoff, Niederalkyl, Niederalkoxy oder unsubstituiertes oder wie angegeben substituiertes Carbamyl, bedeutet und $Y_2$ und $Y_3$ gemeinsam eine zusätzliche Bindung darstellen, oder worin $R'_2$ Cyano oder eine unsubstituierte oder durch Acyl, Niederalkyl oder Niederalkylen bzw. Aza-, Oxa- oder Thianiederalkylen substituierte Carbamylgruppe darstellt, $Y_1$ und $Y_2$ gemeinsam für Imino stehen und $Y_3$ Wasserstoff ist, oder einem Tautomeren und/oder Salz davon umsetzt, erforderlichenfalls ein erhaltenes Isomerengemisch in die Komponenten auftrennt und das Isomere der Formel I isoliert und gewünschtenfalls die verfahrensgemäss erhältliche Verbindung in eine andere Verbindung der Formel I umwandelt.

Tautomere von Verbindungen der Formel III sind beispielsweise Cyanacetyl-Tautomere der Formel $N{\equiv}C{-}CH_2{-}R'_2$ (IIIa) von Ketiminverbindungen der Formel III, worin $Y_1$ und $Y_2$ Imino darstellen und $Y_3$ Wasserstoff ist.

Salze von Verbindungen der Formel III sind beispielsweise Metallsalze, wie Alkalimetallsalze, von Verbindungen der Formel III, worin $Y_1$ und $Y_2$ gemeinsam Imino darstellen und $Y_3$ Wasserstoff ist, bzw. der Tautomeren der Formel IIIa davon.

Die Umsetzung erfolgt in üblicher Weise, vorteilhaft in einem inerten Lösungsmittel, erforderlichenfalls in Gegenwart eines Kondensationsmittels und/oder bei erhöhter Temperatur. Inerte Lösungsmittel sind beispielsweise aromatische oder araliphatische Kohlenwasserstoffe, wie Benzol oder Toluol, oder Aether,

wie Tertiärbutoxymethan, Tetrahydrofuran oder Dioxan, und für die Umsetzung mit Verbindungen der Formel IIIa ferner Alkohole, wie Niederalkanole, z.B. Methanol, Aethanol oder Butanol. Kondensationsmittel sind beispielsweise für die Umsetzung mit Verbindungen der Formel III, worin $Y_1$ und $Y_2$ gemeinsam Imino darstellen und $Y_3$ Wasserstoff ist, bzw. deren Tautomeren der Formel IIIa salzbildende Kondensationsmittel, wie Metallalkoholate, z.B. Natriummethanolat, Natriumäthanolat, oder anderer Alkalimetallniederalkanolate, Metallamide, wie Natriumamid, Lithiumdiisopropylamid und dergleichen, oder metallorganische Verbindungen, wie Niederalkylmagnesiumhalogenide oder Niederalkyllithiumverbindungen, z.B. Methyl- oder Butylmagnesiumbromid oder Butyllithium. Statt in Gegenwart eines der genannten Kondensationsmittel zu arbeiten kann man die Komponente der Formel III bzw. IIIa auch als Salz einsetzen.

Bevorzugte Ausführungsformen dieses Verfahrens sind beispielsweise die Umsetzung eines Azides der Formel II mit einer Verbindung der Formel III, worin $Y_1$ Wasserstoff oder Niederalkyl ist und $Y_2$ und $Y_3$ eine zusätzliche Bindung darstellen, in Benzol oder Dioxan bei etwa 60-120°C, vorzugsweise Siedetemperatur, sowie mit Verbindungen der Formeln IIIa in Gegenwart von Natriummethanolat bzw. Natriumäthanolat und Methanol bzw. Aethanol als Lösungsmittel bei etwa 40 bis 100°C, vorzugsweise bei Siedetemperatur.

Die Ausgangsstoffe der Formel III und teilweise der Formel II sind bekannt. Neue Ausgangsstoffe der Formel II können in Analogie zur Bildungsweise der bekannten erhalten werden, beispielsweise indem man eine Verbindung der Formel Ph-alk-X (IV), worin X reaktionsfähiges verestertes Hydroxy, wie Halogen, z.B. Chlor, Brom oder Jod, oder Sulfonyloxy, wie Niederalkansulfonyloxy, gegebenenfalls substituiertes Benzolsulfonyloxy, wie Methan-, Aethan-, Benzol-, p-Toluol- oder p-Brombenzolsulfonyloxy, oder Fluorsulfonyloxy, bedeutet, mit einem Alkalimetallazid, z.B. mit Natriumazid, umsetzt, beispielsweise in Dimethylsulfoxid oder Dimethylformamid, oder indem

man einen Alkohol der Formel IV (X = Hydroxy), in Gegenwart von Triphenylphosphin und eines Azodicarbonsäureesters, z.B. von Azodicarbonsäurediäthylester, mit Stickstoffwasserstoffsäure zur Reaktion bringt, beispielsweise in Toluol.

Die neuen Verbindungen können ferner hergestellt werden, indem man eine Verbindung der Formel

Ph - alk - Z (IV),

worin Z reaktionsfähiges verestertes Hydroxy bedeutet, mit einem 1H—1,2,3-Triazolderivat der Formel

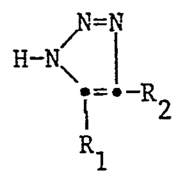

(V)

oder einem Salz davon umsetzt,
erforderlichenfalls ein erhaltenes Isomerengemisch in die Komponenten auftrennt und das Isomere der Formel I isoliert und gewünschtenfalls die verfahrensgemäss erhältliche Verbindung in eine andere Verbindung der Formel I umwandelt.

Reaktionsfähiges verestertes Hydroxy Z ist beispielsweise Halogen, z.B. Chlor, Brom oder Jod, oder Sulfonyloxy, wie Niederalkansulfonyloxy, gegebenenfalls substituiertes Benzolsulfonyloxy, wie Methan-, Aethan-, Benzol-, p-Toluol- oder p-Brombenzolsulfonyloxy, oder Fluorsulfonyloxy.

Salze von Verbindungen der Formel V sind beispielsweise Alkali- oder Erdalkalimetallsalze derselben, wie deren Natrium-, Kalium- oder Calciumsalze.

Die Umsetzung erfolgt in üblicher Weise, beispielsweise in Gegenwart eines basischen Kondensationsmittels oder vorteilhaft, indem

man die Komponente der Formel V als Salz einsetzt, erforderlichenfalls unter Erwärmen , vorzugsweise in einem Lösungs- oder Verdünnungsmittel. Basische Kondensationsmittel sind beispielsweise mit der Komponente der Formel V salzbildende basische Kondensationsmittel, wie Alkalimetallalkoholate, z.B. Natriummethanolat oder Natriumäthanolat, Alkalimetall- oder Erdalkalimetallamide, z.B. Natriumamid oder Lithiumdiisopropylamid. Wie erwähnt wird die Ueberführung der Komponente der Formel V in eines ihrer Salze vorteilhaft vorab durchgeführt, z.B. durch Umsetzung mit einer der genannten Basen. Lösungsmittel sind für die Durchführung der Umsetzung in Gegenwart eines Alkoholates vorzugsweise die entsprechenden Alkohole und bei der Durchführung in Gegenwart von Amiden beispielsweise aprotische organische Lösungsmittel, wie Phosphorsäureniederalkylamide oder Diniederalkylsulfoxide, z.B. Hexamethylphosphorsäuretriamid oder Dimethylsulfoxid.

Das Verfahren eignet sich insbesondere für die Herstellung von Verbindungen, worin eine Aminogruppe $R_1$ bzw. die Aminogruppe als Bestandteil einer Carbamylgruppe $R_2$ mindestens N-mono-, vorzugsweise N,N-disubstituiert ist, d.h. Acylamino, Niederalkylamino oder vorzugsweise Diniederalkyl-, Niederalkylen- bzw. (N'-Niederalkyl)aza-, Oxa- oder Thianiederalkylenamino darstellt.

Die Ausgangsstoffe der Formel IV und V können, sofern sie nicht bereits bekannt sind, in üblicher Weise hergestellt werden. So erhält man Verbindungen der Formel IV z.B., indem man einen entsprechenden Alkohol der Formel IV (Z = Hydroxy) reaktionsfähig verestert, z.B. mittels Thionylchlorid, Phosphortribromid oder eines Sulfonylchlorides. Verbindungen der Formel V können hergestellt werden, indem man Trimethylsilylazid oder Stickstoffwasserstoffsäure mit einer Verbindung der Formel

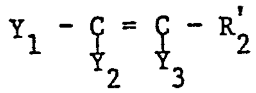 (III),

worin $R_2'$ Cyano oder eine unsubstituierte oder durch Acyl, Niederalkyl oder Niederalkylen bzw. Aza-, Oxa- oder Thianiederalkylen substituierte Carbamylgruppe darstellt, $Y_1$ eine wie angegeben disubstituierte Aminogruppe oder, sofern $R_2'$ für eine unsubstituierte oder durch Acyl, Niederalkyl oder Niederalkylen bzw. Aza-, Oxa- oder Thianiederalkylen substituierte Carbamylgruppe steht, Wasserstoff, Niederalkyl, Niederalkoxy oder unsubstituiertes oder wie angegeben substituiertes Carbamyl, bedeutet und $Y_2$ und $Y_3$ gemeinsam eine zusätzliche Bindung darstellen, oder worin $R_2'$ Cyano oder eine unsubstituierte oder durch Acyl, Niederalkyl oder Niederalkylen bzw. Aza-, Oxa- oder Thianiederalkylen substituierte Carbamylgruppe darstellt, $Y_1$ und $Y_2$ gemeinsam für Imino stehen und $Y_3$ Wasserstoff ist, oder einem Tautomeren und/oder Salz davon umsetzt und gewünschtenfalls in dem erhaltenen 1-Trimethylsilyltriazolderivat eine Aminogruppe $R_1$ und/oder Carbamylgruppe $R_2$ acyliert oder niederalkyliert bzw. durch Niederalkylen oder Aza-, Oxa- oder Thianiederalkylen substituiert und die Silylgruppe durch milde Hydrolyse abspaltet.

Die neuen Verbindungen können ferner hergestellt werden, indem man in einer Verbindung der Formel

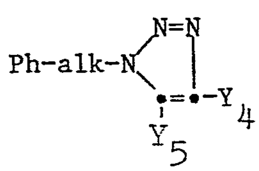 (VI),

worin $Y_4$ einen Rest $Y_A$ und $Y_5$ eine $R_1$-Gruppe oder einen Rest $Y_B$ bedeutet oder $Y_4$ eine $R_2$-Gruppe und $Y_5$ einen Rest $Y_B$ darstellt, wobei $Y_A$ einen in unsubstituiertes oder wie angegeben substituiertes Carbamyl oder, sofern $Y_5$ gegebenenfalls wie angegeben substituiertes Amino $R_1$ darstellt, in Cyano und $Y_B$ einen in gegebenenfalls wie angegeben substituiertes Carbamyl überführbaren Rest bedeutet, oder in einem Salz davon $Y_A$ in Cyano oder gegebenenfalls wie angegeben substituiertes Carbamyl und/oder $Y_B$ in gegebenenfalls wie angegeben substituiertes Carbamyl überführt, erforderlichenfalls ein erhaltenes Isomerengemisch in die Komponenten auftrennt und das Isomere der Formel I isoliert und gewünschtenfalls die verfahrensgemäss erhältliche Verbindung in eine andere Verbindung der Formel I umwandelt.

Reste $Y_A$ und $Y_B$ gemäss der vorstehenden Definition sind beispielsweise freie oder in einer Salz- oder Anhydridform vorliegende Carboxygruppen, gegebenenfalls N-niederalkylierte bzw. durch Niederalkylen bzw. Aza-, Oxa- oder Thianiederalkylen N,N-disubstituierte Amidinogruppen oder veresterte Carboxygruppen, Reste $Y_B$ ferner Cyanogruppen.

Veresterte Carboxygruppen sind beispielsweise mit einem Niederalkanol oder einem Niederalkylmercaptan veresterte Carboxygruppen, d.h. Niederalkoxy- oder Niederalkylthiocarbonylgruppen, können aber auch mit einem beliebigen anderen Alkohol oder Mercaptan, z.B. mit einem gegebenenfalls substituierten Phenol oder Thiophenol, verestert sein.

In Salzform vorliegende Carboxygruppen sind beispielsweise in einer von Ammoniak oder einem Mono- oder Diniederalkylamin abgeleitete Ammoniumsalzform vorliegende Carboxygruppen, ferner in Metall-, z.B. Alkali- oder Erdalkalimetallsalzform vorliegende Carboxygruppen.

In Anhydridform vorliegende Carboxygruppen sind beispielsweise in einer Halogenidform vorliegende Carboxygruppen, wie Chlorcarbonyl, können aber auch mit einer reaktiven Carbonsäure anhydridisiert sein und beispielsweise Alkoxycarbonyloxycarbonyl oder Trifluoracetoxycarbonyl bedeuten.

Gegebenenfalls niederalkylierte bzw. durch Niederalkylen bzw. Aza-, Oxa- oder Thianiederalkylen N,N-disubstituierte Amidinogruppen sind beispielsweise unsubstituierte oder N-mono-, N,N-di- oder N,N'-dinie-

deralkylierte bzw. durch Niederalkylen bzw. Aza-, Oxa- oder Thianiederalkylen N,N-disubstituierte Amidinogruppen.

Die Ueberführung der genannten Gruppen $Y_4$ in Cyano oder gegebenenfalls wie angegeben substituiertes Carbamyl bzw. von $Y_5$ in gegebenenfalls wie angegeben substituiertes Carbamyl erfolgt in üblicher Weise, ausgehend von freien, veresterten oder in einer Anhydridform vorliegenden Carboxygruppen und gegebenenfalls N-niederalkylierten bzw. durch Niederalkylen bzw. Aza-, Oxa- oder Thianiederalkylen N,N-disubstituierten Aminidinogruppen durch Solvolyse, d.h. Hydrolyse oder Ammono- bzw. Aminolyse (Umsetzung mit Ammoniak oder einem Mono- oder Diniederalkylamin, Niederalkylenamin bzw. Aza-, Oxa- oder Thianiederalkylenamin).

Durch Hydrolyse kann man beispielsweise gegebenenfalls niederalkylierte bzw. durch Niederalkylen bzw. Aza-, Oxa- oder Thianiederalkylen N,N-disubstituierte Amidinogruppen $Y_4$ und/oder $Y_5$ in gegebenenfalls entsprechend substituiertes Carbamyl und Cyano $Y_5$ in Carbamyl überführen. Die Hydrolyse wird beispielsweise in Gegenwart eines sauren Hydrolysemittels, wie einer Mineral-, Sulfon- oder Carbonsäure, z.B. von Schwefelsäure, Phosphorsäure, Salzsäure oder einer anderen Halogenwasserstoffsäure, p-Toluolsulfonsäure oder einer anderen organischen Sulfonsäure, oder einer Niederalkansäure, wie Essigsäure, vorzugsweise in katalytischen Mengen, durchgeführt.

Durch Ammono- bzw. Aminolyse kann man beispielsweise freie oder in einer Salzform vorliegende oder veresterte Carboxygruppen in unsubstituiertes oder wie angegeben substituiertes Carbamyl überführen. Dabei arbeitet man erforderlichenfalls in Gegenwart eines Kondensationsmittels, vorteilhaft in einem inerten Lösungsmittel. Kondensationsmittel sind ausgehend von in Anhydridform vorliegendem Carboxy beispielsweise basische Kondensationsmittel, wie Alkalimetallhydroxide oder -carbonate, oder organische Stickstoffbasen, wie der einzuführenden Aminogruppe entsprechende

Amine im Ueberschuss oder tertiäre organische Stickstoffbasen, wie Triniederalkylamine oder tertiäre heteroaromatische Stickstoffbasen, wie Triäthylamin oder Pyridin, und ausgehend von verestertem Carboxy saure Kondensationsmittel, wie Mineralsäuren, wie Halogenwasserstoffsäuren und dergleichen. Freie Carboxygruppen können unter Dehydratisierung der intermediär gebildeten Ammoniumsalze, z.B. durch Erhitzen oder Einwirkung wasserentziehender Mittel, wie von Säureanhydriden, z.B. Phosphorpentoxid, Acetylchlorid und dergleichen, oder von Carbodiimiden, z.B. N,N'-Dicyclohexylcarbodiimid, in gegebenenfalls niederalkyliertes Carbamyl überführt werden.

Die Ausgangsstoffe der Formel VI können, soweit sie nicht bekannt sind, in üblicher Weise hergestellt werden, beispielsweise indem man ein Azid der Formel

$$Ph\text{-}alk\text{-}N_3 \qquad (II)$$

mit einer Verbindung der Formel

$$Y_1-\underset{Y_2}{\underset{|}{C}} = \underset{Y_3}{\underset{|}{C}} - Y_4 \qquad (VII)$$

worin $Y_1$ eine Gruppe $Y_5$ ist und $Y_2$ und $Y_3$ eine zusätzliche Bindung darstellen oder $Y_1$ Wasserstoff oder Niederalkyl, $Y_2$ Hydroxy und $Y_3$ Wasserstoff bedeutet oder $Y_1$ und $Y_2$ gemeinsam Imino darstellen und $Y_3$ Wasserstoff ist, oder einem Tautomeren und/oder Salz davon umsetzt, beispielsweise wie für die Umsetzung mit entsprechenden Verbindungen der Formel III beschrieben.

Ausgangsstoffe der Formel VI, worin $Y_5$ Niederalkoxy und $Y_4$ eine Gruppe $Y_A$ ist, können ferner hergestellt werden, indem man ein Azid der Formel II mit einer Säure der Formel $HOOC\text{-}CH_2\text{-}Y_A$ (XI), oder einem Ester, wie Niederalkylester, davon, z.B. mit einem Malonsäurediniederalkylester, und/oder einem Salz davon umsetzt, vorzugsweise in Gegenwart eines Alkalimetallalkanolates und in dem

gebildeten 5-Hydroxy-1-Phalk-4-$Y_A$-1H-1,2,3-triazol die Hydroxygruppe niederalkyliert, vorzugsweise durch Umsetzung mit einem reaktiven Niederalkylester, wie Dimethylsulfat, in Gegenwart von Natriumhydroxid.

Die neuen Verbindungen können ferner hergestellt werden, indem man aus einer Verbindung der Formel

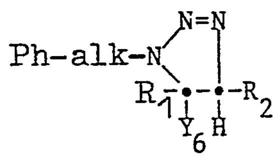

(IX),

worin $Y_6$ einen abspaltbaren Rest bedeutet, H-$Y_6$ abspaltet und erforderlichenfalls ein erhaltenes Isomerengemisch in die Komponenten auftrennt und das Isomere der Formel I isoliert und gewünschtenfalls die verfahrensgemäss erhältliche Verbindung in eine andere Verbindung der Formel I umwandelt.

Abspaltbare Reste $Y_6$ sind beispielsweise Hydroxy-, Niederalkoxy- oder gegebenenfalls substituierte Aminogruppen, wobei Niederalkoxy vorzugsweise mit Niederalkoxy $R_1$ und gegebenenfalls substituiertes Amino vorzugsweise mit freiem oder substituiertem Amino $R_1$ identisch ist.

Die Abspaltung von H-$Y_6$ erfolgt im allgemeinen spontan, in einzelnen Fällen kann die Zufuhr thermischer Energie oder die Gegenwart eines sauren oder basischen Mittels, wie einer Mineralsäure, z.B. von Schwefel- oder Salzsäure, oder einer Carbon- oder Sulfonsäure, z.B. von Essig- oder p-Toluolsulfonsäure, oder eines Alkalimetallhydroxides oder -alkoholates, z.B. von Natriummethanolat, vorteilhaft sein.

Die Ausgangsstoffe der Formel IX werden vorzugsweise in situ hergestellt und ohne Isolierung weiterumgesetzt, beispielsweise indem man eine Verbindung der Formel

$$\text{Ph - alk - } N_3 \qquad \text{(II)}$$

mit einer Verbindung der Formel

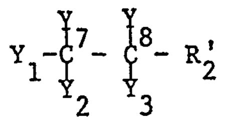 (X),

worin $R_2'$ Cyano oder eine unsubstituierte oder durch Acyl, Niederalkyl oder Niederalkylen bzw. Aza-, Oxa- oder Thianiederalkylen substituierte Carbamylgruppe darstellt, $Y_1$ und $Y_2$ Niederalkoxy oder gegebenenfalls substituiertes Amino $R_1$, $Y_3$ Wasserstoff und $Y_7$ und $Y_8$ eine zusätzliche Bindung oder $Y_1$, $Y_2$ und $Y_7$ Niederalkoxy und $Y_3$ und $Y_8$ Wasserstoff oder $Y_1$ und $Y_2$ gemeinsam Oxo, $Y_7$ Wasserstoff oder Niederalkyl und $Y_3$ sowie $Y_8$ Wasserstoff bedeuten oder einem Tautomeren und/oder Salz davon, beispeilsweise mit einer Verbindung der Formel $Y_1'-C(=O)-CH_2-R_2'$ (Xa; $Y_1'$ = Wasserstoff oder Niederalkyl), $Y_1-C(Y_2)=CH-R_2'$ (Xb; $Y_1$ und $Y_2$= Niederalkoxy oder unabhängig voneinander unsubstituiertes oder wie für $R_1$ angegeben substituiertes Amino), oder $Y_1-C(Y_2)(Y_7)-CH_2-R_2'$ (Xc; $Y_1$, $Y_2$ und $Y_7$ = Niederalkoxy), umsetzt.

Verbindungen der Formel I, worin $R_1$ Niederalkoxy bedeutet, können ferner hergestellt werden, indem man in einer Verbindung der Formel

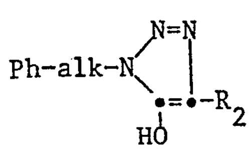 (XII)

die Hydroxygruppe in Niederalkoxy überführt und erforderlichenfalls ein erhaltenes Isomerengemisch in die Komponenten auftrennt und das Isomere der Formel I isoliert und gewünschtenfalls die verfahrensgemäss erhältliche Verbindung in eine andere Verbindung der Formel I umwandelt.

Die Ueberführung von Hydroxy in Niederalkoxy erfolgt beispielsweise durch Umsetzung mit einem reaktiven Niederalkylester, wie einem Halogenwasserstoff-, wie Chlor-, Brom- oder Jodwasserstoffsäureester, einem Schwefelsäureester, d.h. einem Diniederalkylsulfat, oder einem Sulfonsäureester, wie einem Niederalkan- oder gegebenenfalls substituierten Benzolsulfonsäureester, z.B. Methan-, Benzol, p-Toluol- oder p-Brombenzolsulfonsäureester eines Niederalkanols, vorteilhaft in Gegenwart eines basischen Mittels, wie eines Alkalimetallhydroxides, -carbonates oder -alkoholates, z.B. von Natrium- oder Kaliumhydroxid, Kaliumcarbonat oder Natriummethanolat, oder einer tertiären organischen Stickstoffbase, wie eines Triniederalkylamins, z.B. von Triäthylamino, oder von Pyridin.

Die Ausgangsstoffe der Formel XII können beispielsweise erhalten werden, indem man ein Azid der Formel

$$Ph - alk - N_3 \qquad (II)$$

mit einer Säure der Formel $HOOC-CH_2-R_2$ (XI) oder einem Ester, z.B. einem Niederalkylester, davon umsetzt, z.B. wie für die Umsetzung mit Verbindungen der Formel IIIa angegeben.

Verfahrensgemäss erhältliche Verbindungen können in andere Verbindungen der Formel I überführt werden, indem man eine oder mehrere Variablen der allgemeinen Formel I in andere überführt.

So kann man Cyano $R_2$ zu Carbamyl hydrolysieren, beispielsweise unter basischen Bedingungen, wie in Gegenwart eines Alkalimetallhydroxides. Cyano kann aber auch in N-Niederalkyl- bzw. N,N-Diniederalkyl-carbamyl überführt werden, beispielsweise indem man das Nitril der Formel I zunächst, z.B. durch Behandeln mit einem Niederalkanol, Phenol oder einem beliebigen anderen Alkohol in einen Iminoäther überführt, diesen mit einem Mono- oder Diniederalkylamin umsetzt,

und das gebildete Amidin hydrolysiert, vorzugsweise unter mild-sauren Bedingungen.

Umgekehrt kann man Carbamyl $R_2$ zu Cyano dehydratisieren, beispielsweise durch Behandeln mit einem wasserentziehenden Mittel, z.B. einem Säureanhydrid, wie Phosphorpentoxid oder Phosphoroxychlorid, mit einem cyclischen Silazan oder mit Dichlorcarben bzw. mit Trichlormethan in Gegenwart von etwa 40%-iger Natronlauge und eines Phasentransfer-Katalysators, z.B. vor Benzyl-trimethylammoniumchlorid.

Weiterhin kann man primäres Amino $R_1$ in Acylamino, Mono- oder Diniederalkylamino oder Niederalkylen- bzw. Aza-, Oxa- oder Thianiederalkylenamino, ebenso Mononiederalkylamino $R_1$ in Diniederalkylamino überführen. Die Acylierung erfolgt z.B. durch Umsetzung mit einem entsprechenden Säureanhydrid bzw. -chlorid, wie Acetanhydrid, dem gemischten Anhydrid von Essig- und Ameisensäure, einem Chlor- oder Bromameisensäureniederalkylester, Methansulfonylchlorid oder Dimethylcarbamoylchlorid, erforderlichenfalls in Gegenwart einer Base, z.B. von Triäthylamin oder Pyridin, oder einer Säure, z.B. von Schwefelsäure. Die Niederalkylierung erfolgt z.B. mit einem reaktiven Ester eines Niederalkandiols bzw. Aza-, Oxa- oder Thianiederalkandiols, wie einem Niederalkyl(endi)-halogenid, z.B. -bromid oder -jodid, Niederalkyl(endi)sulfonat, z.B. -methansulfonat oder -p-toluolsulfonat, oder einem Diniederalkylsulfat, z.B. Dimethylsulfat, vorzugsweise unter basischen Bedingungen,wie in Gegenwart von Natronlauge oder Kalilauge, und vorteilhaft eines Phasentransfer-Katalysators, wie Tetrabutylammoniumbromid oder Benzyltrimethylammoniumchlorid. In ganz analoger Weise kann man auch Carbamyl $R_2$ und/oder $R_1$ in N-Acylcarbamyl, N-Mono- oder N,N-Diniederalkylcarbamyl bzw. N,N-Niederalkylencarbamyl oder N-N-(Aza-, Oxa- oder Thia)-niederalkylencarbamyl, ebenso N-Mononiederalkylcarbamyl in N,N-Diniederalkylcarbamyl überführen, wobei indes stärker basische Kondensationsmittel, wie Alkalimetallamide oder -alkoholate, z.B. Natriumamid oder Natriummethanolat, erforderlich sein können.

Die neue Verbindung kann, je nach der Wahl der Ausgangsstoffe und Arbeitsweisen, in Form eines der möglichen Isomeren, z.B. bezüglich der Stellung von $R_1$ und $R_2$, oder als Gemische derselben, je nach der Anzahl der asymmetrischen Kohlenstoffatome ferner als optische Isomere, wie Antipoden, oder als Gemische derselben, wie Racemate, Diastereoisomerengemische oder Racematgemische, vorliegen.

Erhaltene Gemische an Stellungsisomeren sowie Diastereomerengemische und Racematgemische können auf Grund der physikalisch-chemischen Unterschiede der Bestandteile in bekannter Weise in die reinen Stellungsisomeren, Diastereomeren bzw. Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung eines sauren Vorproduktes, z.B. der Formel VI ($Y_4$ und/oder $Y_5$ = Carboxyl), mit einem mit der racemischen Säure Ester bildenden optisch aktiven Alkohol und Trennung der auf diese Weise erhaltenen Ester, z.B. auf Grund ihrer verschiedenen Löslichkeiten, in die Diastereomeren, aus denen die Antipoden durch Einwirkung geeigneter Mittel freigesetzt werden können, zerlegt. Vorteilhaft isoliert man den wirksameren der beiden Antipoden.

Racemate salzbildender Verbindungen der Formel I können auch durch Umsetzung mit einer optisch aktiven Hilfsverbindung in Diastereomerengemische, z.B. mit einer optisch aktiven Säure in Gemische diastereomerer Säureadditionssalze, und Trennung derselben in die Diastereomeren, aus denen die Enantiomeren in der jeweils üblichen Weise freigesetzt werden können, gespalten werden.

Für diesen Zweck übliche optisch aktive Säuren sind z.B. D- oder L-Weinsäure, Di-o-Toluylweinsäure, Aepfelsäure, Mandelsäure, Camphersulfonsäuren oder Chinasäure.

Ferner können erhaltene freie salzbildende Verbindungen in an sich bekannter Weise in Säureadditionssalze überführt werden, z.B. durch Umsetzen einer Lösung der freien Verbindung in einem geeigneten Lösungsmittel oder Lösungsmittelgemisch mit einer der vorgenannten Säuren oder mit einer Lösung davon, oder mit einem geeigneten Anionenaustauscher.

Erhaltene Säureadditonssalze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder mit einem geeigneten Anionenaustauscher.

Erhaltene Säureadditonssalze können in an sich bekannter Weise in andere Säureadditionssalze überführt werden, z.B. durch Behandlung eines Salzes einer organischen Säure mit einem geeigneten Metallsalz, wie einem Natrium-, Barium- oder Silbersalz, einer Säure in einem geeigneten Lösungsmittel, in welchem ein sich bildendes anorganisches Salz unlöslich ist und damit aus dem Reaktionsgemisch ausscheidet.

Die Verbindungen , einschliesslich ihrer Salze können auch in Form der Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn-

und zweckmässig gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

So können sämtliche Zwischenprodukte der Formel IX gemäss der Verfahrensvariante der Umsetzung von Verbindungen der Formeln II und X in situ gebildet und ohne Isolierung weiterumgesetzt werden.

Neue Ausgangsstoffe der Formeln II, V, VI, IX und XII, die speziell für die Herstellung der erfindungsgemässen Verbindungen entwickelt wurden, insbesondere die zu den eingangs als bevorzugt gekennzeichneten Verbindungen der Formel I führende Ausgangsstoffauswahl, die Verfahren zu ihrer Herstellung sowie ihre Verwendung als Zwischenprodukte bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Laktose, Dextrose, Saccharose, Mannitol, Sorbitol, Cellulose und/oder Glycin, und/oder Schmiermittel, z.B. Kieselerde, Talk, Stearinsäure oder Salze davon, wie Magnesium- oder Calciumstearat, und/oder Polyäthylenglykol, aufweisen. Tabletten können ebenfalls Bindemittel, z.B. Magnesiumaluminiumsilikat,

Stärken, wie Mais-, Weizen-, Reis- oder Pfeilwurzstärke, Gelatine, Traganth, Methylcellulose, Natriumcarboxylmethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel aufweisen. Ferner kann man die neue Verbindung der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes.

Die Erfindung betrifft ebenfalls die Verwendung der Verbindungen der Formel I, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 5 und etwa 50 mg/kg und für Warmblütter mit einem Gewicht von etwa 70 kg vorzugsweise etwa 0,5 g und etwa 5,0 g.

Die folgenden Beispiele dienen zur Illustration der Erfindung; Temperaturen werden in Celsiusgraden angegeben.

Beispiel 1: 23 g (1 Mol) Natrium werden in 1 l Aethylalkohol gelöst und darauf 101 g (1,2 Mol) Cyanacetamid zugegeben. Zu dieser Suspension gibt man bei 40-50° 167,5 g (1 Mol) o-Chlorbenzylazid und erhitzt 2 Stunden zum Rückfluss. Nach Abkühlen auf 30° gibt man 1000 ml Wasser hinzu, saugt das ausgefallene Produkt ab und wäscht mehrmals mit warmem Wasser nach. Nach Umkristallisation aus Dioxan und Toluol erhält man so das 5-Amino-1-(o-chlorbenzyl)-1H-1,2,3-triazol-4-carboxamid in Form farbloser Kristalle vom Smp. 206°-207°.

Beispiel 2: In analoger Weise erhält man ausgehend von 2,6-Dichlorbenzylazid das 5-Amino-1-(2,6-dichlorbenzyl)-1H-1,2,3-triazol-4-carboxamid, Smp. 243-245° (nach Kristallisation aus Methanol).

Das Ausgangsmaterial kann z.B. folgendermassen hergestellt werden.

Zu einer Suspension von 7,2 g (0,11 Mol) Natriumazid in 50 ml Dimethylsulfoxyd gibt man bei Raumtemperatur 24 g (0,1 Mol) 2,6-Dichlorbenzylbromid in 50 ml Dimethylsulfoxyd hinzu und lässt 2 Stunden bei Raumtemperatur rühren. Dann wird mit 250 ml Wasser verdünnt, mit Cyclohexan extrahiert und die organische Phase mehrmals mit Wasser gewaschen. Nach Trocknen über Natriumsulfat destilliert man das Cyclohexan bei 50° im Vakuum ab. Man erhält das 2,6-Dichlorbenzylazid als farblose Flüssigkeit. Es wird ohne weitere Reinigung eingesetzt.

Beispiel 3: In analoger Weise wie in Beispiel 2 beschrieben erhält man ausgehend von o-Methylbenzylchlorid über α-Azido-o-xylol das 5-Amino-1-(o-methylbenzyl)-1H-1,2,3-triazol-4-carboxamid, Smp. 227-228° (nach Kristallisation aus Dioxan/Aethanol).

Beispiel 4: In analoger Weise wie in Beispiel 1 beschrieben erhält man ausgehend von o-Fluorbenzylazid das 5-Amino-1-(o-fluorbenzyl)-1H-1,2,3-triazol-4-carboxamid, Smp. 189-190° (aus Methanol).

Beispiel 5: In analoger Weise wie in Beispiel 1 beschrieben erhält man ausgehend von o-Brombenzylazid das 5-Amino-1-(o-brombenzyl)-1H-1,2,3-triazol-4-carboxamid, Smp. 211-213° (aus Aethanol).

Beispiel 6: In analoger Weise wie in Beispiel 1 beschrieben erhält man ausgehend von o-Trifluormethylbenzylazid das 5-Amino-1-(o-trifluormethylbenzyl)-1H-1,2,3-triazol-4-carboxamid, Smp. 197-198° (aus Methanol).

Das Ausgangsmaterial kann z.B. folgendermassen hergestellt werden:

Zu einer Lösung von 26,2 g (0,1 Mol) Triphenylphosphin in 260 ml Toluol tropft man bei 10-20° zunächst eine Lösung von 17,4 g (0,1 Mol) Azodicarbonsäurediäthylester in 50 ml Toluol und dann bei 5-10° eine Lösung von 17,6 g (0,1 Mol) o-Trifluormethylbenzylalkohol in 120 ml einer 1-normalen Lösung von Stickstoffwasserstoffsäuren in Toluol hinzu und lässt 2 Stunden bei Raumtemperatur rühren. Der ausgefallene Hydrazino-dicarbonsäureester wird abgesaugt, die Toluollösung eingedampft und der Rückstand mit Cyclohexan behandelt. Die Cyclohexanlösung wird von den unlöslichen Teilen abdekantiert, durch wenig Kieselgel filtriert und bei 50° im Vakuum eingedampft. Man erhält so das o-Trifluormethyl-benzylazid als farblose Flüssigkeit.

Beispiel 7: In analoger Weise wie in Beispiel 6 beschrieben erhält man ausgehend von 2,3-Dimethylbenzylalkohol über 2,3-Dimethylbenzylazid das 5-Amino-1-(2,3-dimethylbenzyl)-1H-1,2,3-triazol-4-carboxamid, Smp. 217°-219° (aus Essigsäureäthylester).

Beispiel 8: In analoger Weise wie in Beispiel 2 beschrieben erhält man ausgehend von 1-(o-Chlorphenyl)-phenäthylchlorid über 1-(o-Chlorphenyl)-äthylazid das 5-Amino-1-[1-(o-Chlorphenyl)-äthyl]-1H-1,2,3-triazol-4-carboxamid, Smp. 202°-204° (aus Aethanol).

Beispiel 9: In analoger Weise wie in Beispiel 2 beschrieben erhält man ausgehend von 1-(o-Chlorphenyl)-propylchlorid über 1-(o-Chlorphenyl)-propylazid das 5-Amino-1-[1-(o-chlorphenyl)-propyl]-1H-1,2,3-triazol-4-carboxamid, Smp. 152-153° (aus Aethanol).

Beispiel 10: In analoger Weise wie in Beispiel 6 beschrieben erhält man ausgehend von 1-(o-Chlorphenyl)-butanol über 1-(o-Chlorphenyl)-butylazid das 5-Amino-1-[1-(o-chlorphenyl)-butyl]-1H-1,2,3-triazol-4-carboxamid, Smp. 150-152° (aus Methanol).

Beispiel 11: 25,2 g (0,1 Mol) 5-Amino-1-(o-chlorbenzyl)-1H-1,2,3-triazol-4-carboxamid werden in 100 ml Dimethylformamid gelöst und unter Rühren bei 0° tropfenweise mit 30,6 g (0,2 Mol) Phosphoroxychlorid versetzt. Dann wird rasch aufgeheizt, bis das Reaktionsgemisch 80° erreicht hat und dann sofort wieder auf 20° abgekühlt. Bei dieser Temperatur werden 100 ml n-Salzsäure zugetropft, anschliessend wird rasch aufgeheizt und 5 Minuten zum Rückfluss erhitzt. Die heisse Reaktionslösung wird mit 400 ml Wasser verdünnt und das ausgefallene Produkt abgesaugt. Nach mehrmaligem Waschen mit Wasser und anschliessender Kristallisation aus Aethylalkohol erhält man so das 5-Amino-1-(o-chlorbenzyl)-1H-1,2,3-triazol-4-carbonsäurenitril, Smp. 174-176°.

Beispiel 12: Eine Lösung von 11,7 g (0,05 Mol) 5-Amino-1-(o-chlorbenzyl)-1H-1,2,3-triazol-4-carbonsäurenitril und 21,3 g (0,15 Mol) Methyljodid in 250 ml Acetonitril wird bei 20-25° in eine gerührten Suspension von 11,2 g pulverisiertem Kaliumhydroxyd und 1,6 g (0,005 Mol) Tetrabutylammoniumbromid in 100 ml Acetonitril eingetropft. Nach 1 Stunde dekantiert man vom anorganischen Material ab und verdampft das Acetonitril im Vakuum. Der Rückstand wird in Diäthyläther gelöst, vom ungelösten Material abfiltriert und die Aetherlösung durch Kieselgel filtriert. Nach Verdampfen des Aethers erhält man das 1-(o-Chlorbenzyl)-5-dimethylamino-1H-1,2,3-triazol-4-carbonsäurenitril vom Smp. 64-67°.

Beispiel 13: Zu einer Lösung von 13,1 g (0,05 Mol) 1-(o-Chlorbenzyl)-5-dimethylamino-1H-1,2,3 -triazol-4-carbonsäurenitril in 300 ml Aethanol und 25 ml 5n Natronlauge werden bei 40°-50° 25 ml 30%ige Wasserstoffperoxyd-Lösung zugetropft. Nach beendetem Zutropfen wird noch 2 Stunden bei 40-50° gerührt, dann mit 600 ml Wasser verdünnt und das ausgefallene Produkt abgesaugt. Nach Waschen mit Wasser und Kristallisation aus 70%igem Aethanol erhält man das 1-(o-Chlorbenzyl)-5-dimethylamino-1H-1,2,3-triazol-4-carboxamid, Smp. 164-166°.

Beispiel 14: 93 g (0,03 Mol) 1-(o-Chlorbenzyl)-1H-1,2,3-triazol-4,5-dicarbonsäure-dimethylester werden in 250 ml Methanol gelöst und bei 50° mit Ammoniak gesättigt. Nach 3 Tagen Stehenlassen bei 60° wird abgekühlt, das auskristallisierte Produkt abgesaugt und mit Methanol gewaschen. Man erhält so das 1-(o-Chlorbenzyl)-1H-1,2,3-triazol-4,5-dicarboxamid vom Smp. 222-224°.

Das Ausgangsmaterial kann z.B. folgendermassen hergestellt werden:

Zu einer am Rückfluss siedenden Lösung von 8,4 g (0,05 Mol) o-Chlorbenzylazid in 50 ml Benzol wird eine Lösung von 8 g (0,054 Mol) Acetylendicarbonsäuredimethylester in 25 ml Benzol zugetropft. Nach

einer weiteren Stunde am Rückfluss wird mit 75 ml Cyclohexan verdünnt und nach Abkühlen die auskristallisierte Substanz abgesaugt. Nach Waschen mit einem Gemisch von Diäthyläther und Hexan (1:1) erhält man so den 1-(o-Chlorbenzyl)-1H-1,2,3-triazol-4,5-dicarbonsäure-dimethylester vom Smp. 88-91°.

Beispiel 15: 2,7 g (10 mM) 1-(o-Chlorbenzyl)-5-methoxy-1H-1,2,3-triazol-4-carbonsäure werden mit 15 ml Thionylchlorid 30 Minuten zum Rückfluss erhitzt. Das überschüssige Thionylchlorid wird bei 60° im Vakuum abdestilliert und das zurückbleibende 1-(o-Chlorbenzyl)-5-methoxy-1H-1,2,3-triazol-4-carbonsäurechlorid in 20 ml Toluol gelöst. Diese Toluollösung wird bei 0-5° zu 20 ml einer konzentrierten wässrigen Ammoniaklösung getropft. Das ausgefallene Produkt wird abgesaugt und mehrmals mit Wasser gewaschen. Man erhält das 1-(o-Chlorbenzyl)-5-methoxy-1H-1,2,3-triazol-4-carboxamid vom Smp. 185-187°.

Das Ausgangsmaterial kann z.B. folgendermassen hergestellt werden:

Zu einer Lösung von 1,15 g (50 mM) Natrium in 50 ml Aethanol gibt man eine Mischung von 8,4 g (50 mM) o-Chlorbenzylazid, 8 g (50 Mol) Malonsäurediäthylester in 25 ml Aethanol hinzu und lässt 20 Stunden bei Raumtemperatur stehen.

Dann werden bei 10-20° 6,3 g (50 mM) Dimethylsulfat zugetropft und nach einer Stunde bei 25° im Vakuum eingedampft. Der Rückstand wird in Aethylacetat gelöst, mit n-Natronlauge und dann mit Wasser gewaschen und erneut eingedampft. Das zurückbleibende Oel wird mit 50 ml Diäthyläther zur Kristallisation gebracht. Man erhält so den 1-(o-Chlorbenzyl)-5-methoxy-1H-1,2,3-triazol-4-carbonsäureäthylester vom Smp. 118-120°.

3,3 g (11,2 mM) 1-(o-Chlorbenzyl)-5-methoxy-1H-1,2,3-triazol-4-carbonsäureäthylester werden in 50 ml warmem Aethanol gelöst und nach Zugabe von 15 ml n-Natronlauge 1 Stunde zum Rückfluss erhitzt. Das ausgefallene Natriumsalz wird durch Zusatz von 250 ml Wasser in Lösung gebracht und anschliessend mit 15 ml 2n Salzsäure angesäuert. Das ausgefallene Produkt wird abgesaugt und mit Wasser gewaschen. Man erhält so die 1-(o-Chlorbenzyl)-5-methoxy-1H-1,2,3-triazol-4-carbonsäure vom Smp. 130-135° (unter Decarboxylierung).

Beispiel 16: 6,4 g (27 mM) 1-(o-Chlorbenzyl)-1H-1,2,3-triazol-4-carbonsäure werden mit 50 ml Thionylchlorid 1 Stunde zum Rückfluss erhitzt. Dann destilliert man das überschüssige Thionylchlorid im Vakuum, löst das zurückbleibende 1-(o-Chlorbenzyl)-1H-1,2,3-triazol-4-carbonsäurechlorid in 50 ml Toluol und tropft diese Lösung bei 5-10° zu 50 ml einer konzentrierten wässrigen Ammoniaklösung. Das ausgefallene Produkt wird abgesaugt, mit Wasser gewaschen und aus Dioxan/Aethanol umkristallisiert. Man erhält so das 1-(o-Chlorbenzyl)-1H-1,2,3-triazol-4-carboxamid vom Smp. 237-239°.

In analoger Weise kann man auch das 1-(o-Chlorbenzyl)-1H-1,2,3-triazol-5-carboxamid vom Smp. 155-158° herstellen.

Die Ausgangsmaterialien können z.B. folgendermassen hergestellt werden:

Eine Lösung von 16,75 g (100 mM) o-Chlorbenzylazid, 7,35 g (100 mM) Propincarbonsäure und 200 ml Toluol wird 24 Stunden bei 50° gerührt. Das ausgefallene Produkt wird nach dem Abkühlen auf Raumtemperatur abgesaugt und zunächst mit Toluol und dann mit Diäthyläther gewaschen. Man erhält so die 1-(o-Chlorbenzyl)-1H-1,2,3-triazol-4-carbonsäure vom Smp. 175° (Zers.). Nach Eindampfen der Filtrate und Waschen des Eindampfrückstandes mit wenig Toluol bleibt die 1-(o-Chlorbenzyl)-1H-1,2,3-triazol-5-carbonsäure vom Smp. 120° (Zers.) zurück.

Beispiel 17: Ein Gemisch von 25,2 g (100 mM) 1-(o-Chlorbenzyl)-5-methyl-1H-1,2,3-triazol-4-carbonsäure und 100 ml Thionylchlorid wird 30 Minuten zum Rückfluss erhitzt. Das überschüssige Thionylchlorid wird darauf im Vakuum abgezogen und das zurückbleibende 1-(o-Chlorbenzyl)-5-methyl-1H-1,2,3-triazol-4-carbonsäurechlorid in 100 ml Toluol gelöst. Diese Lösung wird bei 5-10° zu 100 ml einer konzentrierten wässrigen Ammoniaklösung getropft, das ausgefallene Produkt abgesaugt und mit Wasser gewaschen. Nach Kristallisation aus Aethanol erhält man so das 1-(o-Chlorbenzyl)-5-methyl-1H-1,2,3-triazol-4-carboxamid vom Smp. 181-183°.

Das Ausgangsmaterial kann z.B. folgendermassen hergestellt werden:

Zu einer Lösung von 2,53 g (110 mM) Natrium in 100 ml Aethanol gibt man 14,3 g (110 mM) Acetessigsäureäthylester sowie 16,8 g (100 mM) o-Chlorbenzylazid und erhitzt anschliessend 20 Stunden zum Rückfluss.

Nach Zusatz von 100 ml n-Natronlauge wird weitere 2 Stunden zum Rückfluss erhitzt und dann noch warm mit Salzsäure angesäuert. Das ausgefallene Produkt wird abgesaugt und mit Wasser gewaschen. Nach Kristallisation aus Aethanol erhält man so die 1-(o-Chlorbenzyl)-5-methyl-1H-1,2,3-triazol-4-carbonsäure vom Smp. 186-187° (unter Decarboxylierung).

Beispiel 18: Eine Lösung von 14,7 g (100 mM) o-Methylbenzylazid und 8,3 g (100 mM) But-2-in-carboxamid in 20 ml Dioxan wird 16 Stunden auf 100° erwärmt. Nach dem Verdampfen des Dioxans werden die Isomeren durch Säulenchromatographie getrennt.

Man erhält so das 1-(o-Methylbenzyl)-5-methyl-1H-1,2,3-triazol-4-carboxamid vom Smp. 185-187° (aus Aethanol) sowie ein Nebenprodukt, das verworfen wird.

Beispiel 19: In analoger Weise wie in Beispiel 1 beschrieben erhält man durch Umsetzung von o-Chlorbenzylazid mit 2-Cyan-N-methyl-acetamid das 5-Amino-1-(o-chlorbenzyl)-1H-1,2,3-triazol-4-(N-methyl)-carboxamid vom Smp. 140-142° (aus Acetonitril).

Beispiel 20: In analoger Weise wie in Beispiel 1 beschrieben erhält man durch Umsetzung von o-Chlorbenzylazid mit 2-Cyan-N,N-dimethylacetamid das 5-Amino-1-(o-chlorbenzyl)-1H-1,2,3-triazol-4-(N,N-dimethyl)-carboxamid vom Smp. 143-145° (aus Acetonitril).

Beispiel 21: In analoger Weise wie in Beispiel 2 beschrieben erhält man durch Umsetzung von 2,3-Dichlorbenzylazid mit Cyanacetamid das 5-Amino-1-(2,3-dichlorbenzyl)-1H-1,2,3-triazol-4-carboxamid, Smp. 224-226° (aus Aethanol). Das Ausgangsmaterial kann ausgehend von 2,3-Dichlorbenzylalkohol durch Halogenierung, z.B. mit Phosphortribromid und Umsetzung mit Natriumazid hergestellt werden.

Beispiel 22: 10,4 g roher, etwa 65%-iges 1-(o-Chlorbenzyl)-1H-1,2,3-triazol-4,5-dicarbonsäuredimethylester werden in 50 ml einer 4n-Lösung von Ammoniak in Methanol suspendiert und 1 Stunde bei 50° gerührt. Man lässt abkühlen, saugt ab und löst das in Dioxan mässig lösliche 1-(o-Chlorbenzyl)-1H-1,2,3-triazol-4,5-dicarboxamid mit Dioxan aus dem Nutschkuchen heraus. Es fällt beim Einengen und Abkühlen der Dioxanlösung in Form von Kristallen vom Smp. 222-224° aus.

Das Ausgangsmaterial kann z.B. folgendermassen hergestellt werden:

1,15 g (50 mM) Natrium werden in 100 ml Methanol gelöst. Man fügt 9,25 g (50 mM) 1,2,3-Triazol-4,5-dicarbonsäuredimethylester und 8,05 g (50 mM) o-Chlorbenzylchlorid hinzu und erhitzt 24 Stunden zum Sieden. Dann engt man bis zur beginnenden Kristallisation ein, lässt abkühlen und saugt ab. Der Rückstand kann nach den Trocknen ohne Reinigung eingesetzt werden.

Beispiel 23: In analoger Weise wie in Beispiel 6 beschrieben erhält man ausgehend von 2,4,6-Trimethylbenzylalkohol über 2,4,6-Trimethylbenzylazid das 5-Amino-1-(2,4,6-trimethylbenzyl)-1H-1,2,3-triazol-4-carboxamid vom Smp. 195-196° (aus Essigsäureäthylester).

Beispiel 24: In analoger Weise wie in Beispiel 6 beschrieben erhält man ausgehend von 5-Chlor-2-methyl-benzylalkohol über 5-Chlor-2-methyl-benzylazid das 5-Amino-1-(5-chlor-2-methyl-benzyl)-1H-1,2,3-triazol-4-carboxamid vom Smp. 247-248° (Essigsäure).

Beispiel 25: In analoger Weise wie in Beispiel 1 beschrieben erhält man ausgehend von o-Chlorbenzylazid und Cyanessigsäurepiperidid das 5-Amino-1-(o-chlorbenzyl)-1H-1,2,3-triazol-4-carbonsäurepiperidid vom Smp. 138-140° (Aethanol).

Beispiel 26: In analoger Weise wie in Beispiel 2 beschrieben erhält man ausgehend von m-Chlorbenzylchlorid über m-Chlorbenzylazid das 5-Amino-1-(m-chlorbenzyl)-1H-1,2,3-triazol-4-carboxamid vom Smp. 196-198° (aus Aethanol).

Beispiel 27: In analoger Weise wie in Beispiel 2 beschrieben erhält man ausgehend von m-Trifluormethylbenzylchlorid über m-Trifluormethylbenzylazid das 5-Amino-1-(m-trifluormethylbenzyl)-1H-1,2,3-triazol-4-carboxamid vom Smp. 206-207° (aus Aethanol).

Beispiel 28: In analoger Weise wie in Beispiel 16 beschrieben erhält man ausgehend von 11,8 g (50 mMol) 1-(o-Chlorbenzyl)-1H-1,2,3-triazol-4-carbonsäure über das Säurechlorid durch Behandeln desselben mit Piperidin (50 mMol) das 1-(o-Chlorbenzyl)-1H-1,2,3-triazol-4-carbonsäurepiperidid vom Smp. 141-142° (aus Aethanol) sowie durch Behandeln mit Dimethylamin (50 mMol) das 1-(o-Chlorbenzyl)-1H-1,2,3-triazol-4-carbonsäure-N,N-dimethylamid vom Smp. 120-121° (aus Aethanol).

Beispiel 29: In analoger Weise wie in Beispiel 1 beschrieben erhält man ausgehend von m-Methylbenzylazid das 5-Amino-1-(m-methylbenzyl)-1H-1,2,3-triazol-4-carboxamid vom Smp. 200-202° (aus Aethanol).

Beispiel 30: In analoger Weise wie in Beispiel 16 beschrieben erhält man ausgehend von m-Chlorbenzylazid über die 1-(m-Chlorbenzyl)-1H-1,2,3-triazol-4-carbonsäure vom Smp. 174-176° (Zers.) das 1-(m-Chlorbenzyl)-1H-1,2,3-triazol-4-carboxamid vom Smp. 223-224° (aus Aethanol).

Beispiel 31: In analoger Weise wie in Beispiel 16 beschrieben erhält man ausgehend von m-Trifluormethylbenzylazid über die 1-(m-Trifluormethylbenzyl)-1H-1,2,3-triazol-4-carbonsäure vom Smp. 171° (Zers.) das 1-(m-Trifluormethylbenzyl)-1H-1,2,3-triazol-4-carbonsäureamid vom Smp. 193-195° (aus Aethanol).

Beispiel 32: In analoger Weise wie in Beispiel 1 beschrieben erhält man durch Umsetzung von o-Chlorbenzylazid und Cyanessigsäure-(4-methyl)-piperazid das 5-Amino-1-(o-chlorbenzyl)-1H-1,2,3-triazol-4-carbonsäure-(4-methyl)-piperazid vom Smp. 114-116°. Das Methansulfonat schmilzt bei 208-210° (aus Aceton).

Beispiel 33: In ein auf 60° erwärmtes Gemisch von 30,7 g Acetanhydrid und 0,1 ml Schwefelsäure werden unter Rühren portionsweise 10,1 g 5-Amino-1-(o-chlorbenzyl)-1H-1,2,3-triazol-4-carboxamid eingetragen. Man lässt 30 Minuten bei 60-70° nachrühren, giesst in 250 ml Aethanol, erhitzt 30 Minuten zum Rückfluss, dampft unter vermindertem Druck zur Trockene ein und digeriert mit 250 ml Essigester. Der alsbald gebildete kristalline Niederschlag wird abgesaugt und aus Essigester umkristallisert. Man erhält das 5-Acetylamino-1-(o-chlorbenzyl)-1H-1,2,3-triazol-4-carboxamid vom Smp. 181-183°.

Die Essigester-Mutterlaugen werden vereinigt und zur Trockene eingedampft. Der Rückstand wird an einer Kieselgelsäule mit Toluol/Essigester als Laufmittel chromatographisch gereinigt. Das Roheluat wird eingedampft und aus Toluol umkristallisiert. Man erhält das 5-Acetylamino-1-(o-chlorbenzyl)-1H-1,2,3-triazol-4-(N-acetyl)carboxamid vom Smp. 140-142°.

Beispiel 34: In analoger Weise wie in den Beispielen 1-33 beschrieben kann man ferner herstellen:

1-(o-Chlorbenzyl)-5-formylamino-1H-1,2,3-triazol-4-carboxamid,
1-(o-Chlorbenzyl)-5-formylamino-1H-1,2,3-triazol-4-(N-formyl)-carboxamid,
5-Aethoxycarbonylamino-1-(o-chlorbenzyl)-1H-1,2,3-triazol-4-carboxamid,
1-(o-Chlorbenzyl)-5-(3,3-dimethylureido)-1H-1,2,3-triazol-4-carboxamid,
1-(o-Chlorbenzyl)-5-(N',N'-dimethylglycylamino)-1H-1,2,3-triazol-4-carboxamid,
5-Aethoxyacetylamino-1-(o-chlorbenzyl)-1H-1,2,3-triazol-4-carboxamid,
1-(o-Chlorbenzyl)-5-(4-methylpiperazinocarbonylamino)-1H-1,2,3-triazol-4-carboxamid.

Beispiel 35: Tabletten, enthaltend je 50 mg 5-Amino-1-(o-methylbenzyl)-1H-1,2,3-triazol-4-carboxamid, können wie folgt hergestellt werden.

Zusammensetzung (10000 Tabletten)

| | |
|---|---|
| Wirkstoff | 500.0 g |
| Lactose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdisper.) | 20.0 g |
| Aethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 36: Lacktabletten, enthaltend je 100 mg 5-Amino-1-(o-methylbenzyl)-1H-1,2,3-triazol-4-carboxamid, können wie folgt hergestellt werden:

| Zusammensetzung (für 1000 Tabletten) | |
|---|---|
| Wirkstoff | 100.00 g |
| Lactose | 100.00 g |
| Maisstärke | 70.00 g |
| Talk | 8.50 g |
| Calciumstearat | 1.50 g |
| Hydroxypropyl-methylcellulose | 2.36 |
| Schellack | 0.64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen) befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 mg.

Beispiel 37: In analoger Weise wie in den Beispielen 35 und 36 beschrieben können auch Tabletten bzw. Lacktabletten enthaltend eine andere Verbindung gemäss der Beispiele 1-32 hergestellt werden.

Beispiel 38: Die Verbindung gemäss Beispiel 11 kann auch auf foldendem andern Wege erhalten werden:

1,15 g Natrium werden in 50 ml Aethanol gelöst. Dann gibt man ein Gemisch von 8,4 g o-Chlorbenzylazid, 3,3 g Malodinitril und 50 ml Aethanol hinzu, lässt 24 Stunden bei Raumtemperatur stehen und destilliert das Lösungsmittel unter vermindertem Druck ab. Der Rückstand wird mit 50 ml 2n-Salzsäure und 100 ml Essigsäureäthylester geschüttelt, die organische Phase abgetrennt, eingedampft und der Rückstand zweimal aus Aethanol umkristallisiert. Man erhält so ebenfals das 5-Amino-1-(o-chlorbenzyl)-1H-1,2,3-triazol-4-carbonsäurenitril vom Smp.174-176°.

Patentansprüche für die Vertragsstaaten: BE, DE, CH, LI, FR, IT, LU,NL,SE, GB

1. 1-Phenylniederalkyl-1H-1,2,3-triazolverbindungen der Formel

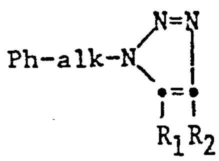

(I),

worin Ph durch Niederalkyl, Halogen und/oder Trifluormethyl substituiertes Phenyl bedeutet, alk Niederalkyliden darstellt, $R_1$ Wasserstoff, Niederalkyl, Niederalkoxy oder eine unsubstituierte oder durch Acyl, Niederalkyl oder Niederalkylen bzw. Aza-, Oxa- oder Thianiederalkylen substituierte Amino- oder Carbamylgruppe bedeutet und $R_2$ für eine unsubstituierte oder durch Acyl, Niederalkyl oder Niederalkylen bzw. Aza-, Oxa- oder Thianiederalkylen substituierte Carbamylgruppe oder, sofern $R_1$ eine unsubstituierte oder durch Acyl, Niederalkyl oder Niederalkylen bzw. Aza-, Oxa- oder Thianiederalkylen substituierte Aminogruppe darstellt, für Cyano bzw., sofern $R_1$ eine unsubstituierte oder durch Acyl, Niederalkyl oder Niederalkylen bzw. Aza-, Oxa- oder Thianiederalkylen substituierte Carbamylgruppe bedeutet, für Wasserstoff oder Niederalkyl steht, und Salze von salzbildenden Verbindungen der Formel I.

2. Verbindungen gemäss Anspruch 1 der Formel I,
worin Ph in 2-Stellung durch Niederalkyl, Halogen oder Trifluormethyl und gegebenenfalls zusätzlich durch Niederalkyl oder Halogen, welches sich beispielsweise in 3-, 4- oder 6-Stellung, vorzugsweise in 3- oder 6-Stellung befindet, substituiertes Phenyl bedeutet, alk Niederalkyliden darstellt, $R_1$ Wasserstoff, Niederalkyl, Niederalkoxy oder eine unsubstituierte oder durch Niederalkyl substituierte Amino- bzw. Carbamylgruppe darstellt und $R_2$ für unsubstituiertes oder durch Niederalkyl substituiertes Carbamyl oder, sofern $R_1$ unsubstituiertes oder durch Niederalkyl substituiertes Amino darstellt, für Cyano steht,

3. Verbindungen gemäss Anspruch 1 der Formel I, worin Ph durch Niederalkyl, Halogen und/oder Trifluormethyl substituiertes Phenyl bedeutet, alk Niederalkyliden darstellt, $R_1$ Wasserstoff, Niederalkyl, Niederalkoxy, Amino, Acylamino, N-Niederalkyl-, N,N-Diniederalkyl-, N,N-Niederalkylen- bzw. N,N-(Aza-, Oxa- oder Thia)-niederalkylenamino, Carbamyl, N-Acylcarbamyl oder N-Niederalkyl-, N,N-Diniederalkyl-, N,N-Niederalkylen- bzw. N,N-(Aza-, Oxa- oder Thia)-niederalkylencarbamyl bedeutet und $R_2$ für Carbamyl, N-Acylcarbamyl, N-Niederalkyl-, N,N-Diniederalkyl-, N,N-Niederalkylen- bzw. N,N-(Aza-, Oxa- oder Thia)-niederalkylencarbamyl oder, sofern $R_1$ Amino, Acylamino, N-Niederalkyl-, N,N-Diniederalkyl-, N,N-Niederalkylen- bzw. N,N-(Aza-, Oxa- oder Thia)-niederalkylenamino ist, für Cyano bzw., sofern $R_1$ Carbamyl, N-Acylcarbamyl, N-Niederalkyl-, N,N-Diniederalkyl-, N,N-Niederalkylen- bzw. N,N-(Aza-, Oxa- oder Thia)-niederalkylencarbamyl bedeutet, für Wasserstoff oder Niederalkyl steht, wobei Acyl jeweils insbesondere Niederalkanoyl, Niederalkoxyniederalkoxycarbonyl, Diniederalkylaminoniederalkanoyl, Niederalkansulfonyl, Niederalkoxycarbonyl, Ureido, 3-Niederalkyl- bzw. 3,3-Diniederalkylureido oder 3,3-Niederalkylen- bzw. 3,3-(Aza-, Oxa- oder Thia)-niederalkylenureido darstellt und Niederalkyl sowie Niederalkyl in N-Niederalkyl- bzw. N,N-Diniederalkylamino bzw. -carbamyl, Niederalkoxy und Niederalkyliden z.B. bis und mit 7 C-Atome und Niederalkylen bzw. Aza-, Oxa- oder Thianiederalkylen z.B. 4 bis und mit 6 Ringglieder aufweisen, oder ein Salz einer salzbildenden Verbindung der vorstehend definierten Art.

4. Verbindungen gemäss Anspruch 2 der Formel I, worin Ph 2-Halogenphenyl, 2-Trifluormethylphenyl, 2-Niederalkylphenyl, 2,3- bzw. 2,6-Dihalogenphenyl, 2-Halogen-3-niederalkyl-phenyl bzw. 3-Halogen-2-niederalkylphenyl, 2-Halogen-6-niederalkyl-phenyl, 2,3- bzw. 2,6-Diniederalkylphenyl, 3-Halogen-2-trifluormethyl-phenyl bzw. 2-Halogen-3-trifluormethylphenyl oder 2-Halogen-6-trifluormethylphenyl bedeutet, alk 1,1-Niederalkyliden darstellt, $R_1$ Wasserstoff, Niederalkyl, Niederalkoxy, Amino, Niederalkylamino, Diniederalkylamino, Carbamyl, N-Niederalkylcarbamyl oder N,N-Diniederalkylcarbamyl bedeutet und $R_2$ Carbamyl, N-Niederalkylcarbamyl oder N,N-Diniederalkylcarbamyl bedeutet, wobei Niederalkyl sowie Niederalkyl in N-Niederalkyl bzw. N,N-Diniederalkylamino bzw. -carbamyl, Niederalkoxy und Niederalkyliden z.B. bis und mit 7 C-Atome aufweisen.

5. Verbindungen gemäss Anspruch 1 der Formel I, worin Ph durch bis und mit 3 Niederalkylgruppen bzw. Halogenatome, Niederalkyl und Halogen oder Trifluormethyl, wobei Niederalkyl mit bis und mit 4 C-Atomen bzw. Halogen die Atomnummer bis und mit 35 aufweist, substituiertes Phenyl bedeutet, alk 1,1-Niederalkyliden mit bis und mit 4 C-Atomen darstellt, $R_1$ Amino, Niederalkanoylamino mit bis und mit 7 C-Atomen oder Niederalkylamino bzw. Diniederalkylamino mit jeweils bis und mit 4 C-Atomen im Alkylteil darstellt und $R_2$ Carbamyl, N-Niederalkanoylcarbamyl mit bis und mit 7 C-Atomen, N-Niederalkyl-, N,N-Diniederalkyl- oder N,N-Niederalkylencarbamyl mit jeweils bis und mit 4 C-Atomen im Alkyl- bzw. 4 bis und mit 6 Ringgliedern im Alkylenteil oder Cyano bedeutet.

6. Verbindungen gemäss Anspruch 2 der Formel I, worin Ph als Niederalkyl- bzw. Halogensubstituenten Niederalkyl mit bis und mit 4 C-Atomen bzw. Halogen der Atomnummer bis und mit 35 aufweisendes 2-Halogenphenyl, 2-Trifluormethylphenyl, 2-Niederalkylphenyl, 2,3- oder 2,6-Dihalogenphenyl oder 2,3- oder 2,6-Diniederalkylphenyl bedeutet, alk 1,1-Niederalkyliden mit bis und mit 4 C-Atomen darstellt, $R_1$ für Amino, Niederalkylamino bzw. Diniederalkylamino mit jeweils bis und mit 4 C-Atomen im Alkylteil bedeutet und $R_2$ Carbamyl bedeutet.

7. 5-Amino-1-(o-chlorbenzyl)-1H-1,2,3-triazol-4-carboxamid gemäss Anspruch 2.

8. 5-Amino-1-(2,6-dichlorbenzyl)-1H-1,2,3-triazol-4-carboxamid, 5-Amino-1-(o-methylbenzyl)-1H-1,2,3-triazol-4-carboxamid, 5-Amino-1-(o-fluorbenzyl)-1H-1,2,3-triazol-4-carboxamid, 5-Amino-1-(o-brombenzyl)-1H-1,2,3-triazol-4-carboxamid, 5-Amino-1-(o-trifluormethylbenzyl)-1H-1,2,3-triazol-4-carboxamid, 5-Amino-1-(2,3-dimethylbenzyl)-1H-1,2,3-triazol-4-carboxamid, 5-Amino-1-[1-(o-chlorphenyl)-äthyl)-1H-1,2,3-triazol-4-carboxamid, 5-Amino-1-[1-(o-chlorphenyl)-propyl]-1H-1,2,3-triazol-4-carboxamid, 5-Amino-1-[1-(o-chlorphenyl)-butyl]-1H-1,2,3-triazol-4-carboxamid, 1-(o-Chlorbenzyl)-5-dimethylamino-1H-1,2,3-triazol-4-carboxamid, 1-(o-Chlorbenzyl)-1H-1,2,3-triazol-4,5-dicarboxamid, 1-(o-Chlorbenzyl)-5-methoxy-1H-1,2,3-triazol-4-carboxamid, 1-(o-Chlorbenzyl)-1H-1,2,3-triazol-4-carboxamid, 1-(o-Chlorbenzyl)-5-methyl-1H-1,2,3-triazol-4-carboxamid, 1-(o-Methylbenzyl)-5-methyl-1H-1,2,3-triazol-4-carboxamid, 5-Amino-1-(o-chlorbenzyl)-1H-1,2,3-triazol-4-(N-methyl)-carboxamid, 5-Amino-1-(o-chlorbenzyl)-1H-1,2,3-triazol-4-(N,N-dimethyl)-carboxamid oder 5-Amino-1-(2,3-dichlorbenzyl)-1H-1,2,3-triazol-4-carboxamid gemäss Anspruch 2.

9. 1-(o-Chlorbenzyl)-1H-1,2,3-triazol-4,5-dicarboxamid, 5-Amino-1-(2,4,6-trimethylbenzyl)-1H-1,2,3-triazol-4-carboxamid, 5-Amino-1-(5-chlor-2-methylbenzyl)-1H-1,2,3-triazol-4-carboxamid, 5-Amino-1-(o-chlorbenzyl)-1H-1,2,3-triazol-4-carbonsäurepiperidid, 5-Amino-1-(m-chlorbenzyl)-1H-1,2,3-triazol-4-carboxamid, 5-Amino-1-(m-trifluormethylbenzyl)-1H-1,2,3-triazol-4-carboxamid, 1-(o-Chlorbenzyl)-1H-1,2,3-triazol-4-carbonsäurepiperidid, 1-(o-Chlorbenzyl)-1H-1,2,3-triazol-4-carbonsäure-N,N-dimethylamid, 5-Amino-1-(m-methylbenzyl)-1H-1,2,3-triazol-4-carboxamid, 1-(m-Chlorbenzyl)-1H-1,2,3-triazol-4-carboxamid, 1-(m-Trifluormethylbenzyl)-1H-1,2,3-triazol-4-carboxamid oder 5-Amino-1-(o-chlorbenzyl)-1H-1,2,3-triazol-4-carbonsäure-(4-methyl)-piperazid oder ein Säureadditionssalz, 5-Acetylamino-1-(o-chlorbenzyl)-1H-1,2,3-triazol-4-carboxamid, 5-Acetylamino-1-(o-chlorbenzyl)-1H-1,2,3-triazol-4-(N-acetyl)carboxamid, 1-(o-Chlorbenzyl)-5-formylamino-1H-1,2,3-triazol-4-carboxamid, 1-(o-Chlorbenzyl)-5-formylamino-1H-1,2,3-triazol-4-(N-formyl)-carboxamid, 5-Aethoxycarbonylamino-1-(o-chlorbenzyl)-1H-1,2,3-triazol-4-carboxamid, 5-Aethoxyacetylamino-1-(o-chlorbenzyl)-1H-1,2,3-triazol-4-carboxamid, 1-(o-Chlorbenzyl)-5-(3,3-dimethylureido)-1H-1,2,3-triazol-4-carboxamid, 1-(o-Chlorbenzyl)-5-(N',N'-dimethylglycylamino)-1H-1,2,3-triazol-4-carboxamid, 1-(o-Chlorbenzyl)-5-(4-methylpiperazinocarbonylamino)-1H-1,2,3-triazol-4-carboxamid, davon gemäss Anspruch 1.

10. Eine Verbindung gemäss einem der Ansprüche 1-9 zur Anwendung in einem Verfahren zur therapeutischen Behandlung des menschlichen oder tierischen Körpers, beispielsweise als antikonvulsives Mittel.

11. Pharmazeutische Präparate enthalten eine Verbindung gemäss einem der Ansprüche 1, 3, 5 und 9 neben üblichen pharmazeutischen Hilfs- und/oder Trägerstoffen.

12. Pharmazeutische Präparate enthalten eine Verbindung gemäss einem der Ansprüche 2, 4 und 6-8 neben üblichen pharmazeutischen Hilfs- und/oder Trägerstoffen.

13. Verfahren zur Herstellung neuer 1-Phenylniederalkyl-1H-1,2,3-triazolverbindungen der Formel

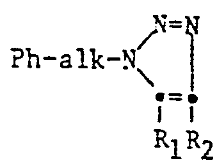

(I),

worin Ph durch Niederalkyl, Halogen und/oder Trifluormethyl substituiertes Phenyl bedeutet, alk Niederalkyliden darstellt, $R_1$ Wasserstoff, Niederalkyl, Niederalkoxy oder eine unsubstituierte oder durch Acyl, Niederalkyl oder Niederalkylen bzw. Aza-, Oxa- oder Thianiederalkylen substituierte Amino- oder Carbamylgruppe bedeutet und $R_2$ für eine unsubstituierte oder durch Acyl, Niederalkyl oder Niederalkylen bzw. Aza-, Oxa- oder Thianiederalkylen substituierte Carbamylgruppe oder, sofern $R_1$ eine unsubstituierte oder durch Acyl, Niederalkyl oder Niederalkylen bzw. Aza-, Oxa- oder Thianiederalkylen substituierte Aminogruppe darstellt, für Cyano bzw., sofern $R_1$ eine unsubstituierte oder durch Acyl,

Niederalkyl oder Niederalkylen bzw. Aza-, Oxa- oder Thianiederalkylen substituierte Carbamylgruppe bedeutet, für Wasserstoff oder Niederalkyl steht, und Salze von salzbildenden Verbindungen der Formel I, dadurch gekennzeichnet, dass man ein Azid der Formel

$$Ph - alk - N_3 \qquad (II)$$

mit einer Verbindung der Formel

$$Y_1 - \underset{Y_2}{\overset{}{C}} = \underset{Y_3}{\overset{}{C}} - R'_2 \qquad (III),$$

worin $R'_2$ Cyano oder eine unsubstituierte oder durch Acyl, Niederalkyl oder Niederalkylen bzw. Aza-, Oxa- oder Thianiederalkylen substituierte Carbamylgruppe darstellt, $Y_1$ eine wie angegeben disubstituierte Aminogruppe oder, sofern $R'_2$ für eine unsubstituierte oder durch Acyl, Niederalkyl oder Niederalkylen bzw. Aza-, Oxa- oder Thianiederalkylen substituierte Carbamylgruppe steht, Wasserstoff, Niederalkyl, Niederalkoxy oder unsubstituiertes oder wie angegeben substituiertes Carbamyl bedeutet und $Y_2$ und $Y_3$ gemeinsam eine zusätzliche Bindung darstellen, oder worin $R'_2$ Cyano oder eine unsubstituierte oder durch Niederalkyl oder Niederalkylen bzw. Aza-, Oxa- oder Thianiederalkylen substituierte Carbamylgruppe darstellt, $Y_1$ und $Y_2$ gemeinsam für Imino stehen und $Y_3$ Wasserstoff ist, oder einem Tautomeren und/oder Salz davon umsetzt oder eine Verbindung der Formel

$$Ph - alk - Z \qquad (IV),$$

worin Z reaktionsfähiges verestertes Hydroxy bedeutet, mit einem 1-H-1,2,3-Triazolderivat der Formel

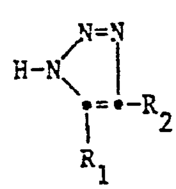

(V)

oder einem Salz davon umsetzt oder in einer Verbindung der Formel

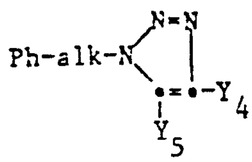

(VI),

worin $Y_4$ einen Rest $Y_A$ und $Y_5$ eine $R_1$-Gruppe oder einen Rest $Y_B$ bedeutet oder $Y_4$ eine $R_2$-Gruppe und $Y_5$ einen Rest $Y_B$ darstellt, wobei $Y_A$ einen in unsubstituiertes oder wie angegeben substituiertes Carbamyl oder, sofern $Y_5$ gegebenenfalls wie angegeben substituiertes Amino $R_1$ darstellt, in Cyano und $Y_B$ einen in gegebenenfalls wie angegeben substituiertes Carbamyl überführbaren Rest bedeutet, oder in einem Salz davon $Y_A$ in Cyano oder gegebenenfalls wie angegeben substituiertes Carbamyl und/oder $Y_B$ in gegebenenfalls wie angegeben substituiertes Carbamyl überführt oder aus einer Verbindung der Formel

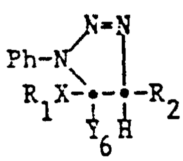

(IX),

worin $Y_6$ einen abspaltbaren Rest bedeutet, $H$-$Y_6$ abspaltet oder in einer Verbindung der Formel

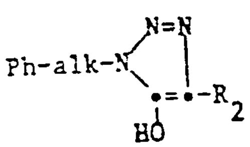

(XII)

die Hydroxygruppe in Niederalkoxy überführt und erforderlichenfalls ein erhaltenes Isomerengemisch in die Komponenten auftrennt und das Isomere der Formel I isoliert und gewünschtenfalls die verfahrensgemäss erhältliche Verbindung in eine andere Verbindung der Formel I umwandelt und/oder ein verfahrensgemäss erhältliche freie Verbindung in ein Salz oder ein verfahrensgemäss erhältliches Salz in die freie Verbindung überführt.

14. Die nach dem Verfahren des Anspruches 13 erhältlichen neuen Verbindungen.

Patentansprüche für den Vertragsstaat AT

1. Verfahren zur Herstellung neuer 1-Phenylniederalkyl-1H-1,2,3-triazolverbindungen der Formel

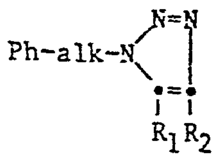 (I),

worin Ph durch Niederalkyl, Halogen und/oder Trifluormethyl substituiertes Phenyl bedeutet, alk Niederalkyliden darstellt, $R_1$ Wasserstoff, Niederalkyl, Niederalkoxy oder eine unsubstituierte oder durch Acyl, Niederalkyl oder Niederalkylen bzw. Aza-, Oxa- oder Thianiederalkylen substituierte Amino- oder Carbamylgruppe bedeutet und $R_2$ für eine unsubstituierte oder durch Acyl, Niederalkyl oder Niederalkylen bzw. Aza-, Oxa- oder Thianiederalkylen substituierte Carbamylgruppe oder, sofern $R_1$ eine unsubstituierte oder durch Acyl, Niederalkyl oder Niederalkylen bzw. Aza-, Oxa- oder Thianiederalkylen substituierte Aminogruppe darstellt, für Cyano bzw., sofern $R_1$ eine unsubstituierte oder durch Acyl, Niederalkyl oder Niederalkylen bzw. Aza-, Oxa- oder Thianiederalkylen substituierte Carbamylgruppe bedeutet, für Wasserstoff oder Niederalkyl steht, und Salze von salzbildenden Verbindungen der Formel I, dadurch gekennzeichnet, dass man ein Azid der Formel

$$Ph - alk - N_3 \qquad (II)$$

mit einer Verbindung der Formel

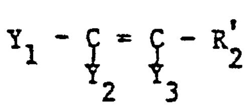 (III),

worin $R_2'$ Cyano oder eine unsubstituierte oder durch Acyl, Niederalkyl oder Niederalkylen bzw. Aza-, Oxa- oder Thianiederalkylen substituierte

Carbamylgruppe darstellt, $Y_1$ eine wie angegeben disubstituierte Aminogruppe oder, sofern $R_2'$ für eine unsubstituierte oder durch Acyl, Niederalkyl oder Niederalkylen bzw. Aza-, Oxa- oder Thianiederalkylen substituierte Carbamylgruppe steht, Wasserstoff, Niederalkyl, Niederalkoxy oder unsubstituiertes oder wie angegeben substituiertes Carbamyl bedeutet und $Y_2$ und $Y_3$ gemeinsam eine zusätzliche Bindung darstellen, oder worin $R_2'$ Cyano oder eine unsubstituierte oder durch Niederalkyl oder Niederalkylen bzw. Aza-, Oxa- oder Thianiederalkylen substituierte Carbamylgruppe darstellt, $Y_1$ und $Y_2$ gemeinsam für Imino stehen und $Y_3$ Wasserstoff ist, oder einem Tautomeren und/oder Salz davon umsetzt oder eine Verbindung der Formel

Ph - alk - Z (IV),

worin Z reaktionsfähiges verestertes Hydroxy bedeutet, mit einem 1-H-1,2,3-Triazolderivat der Formel

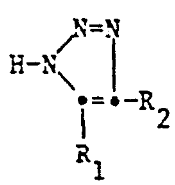

(V)

oder einem Salz davon umsetzt oder in einer Verbindung der Formel

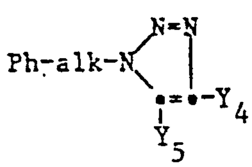

(VI),

worin $Y_4$ einen Rest $Y_A$ und $Y_5$ eine $R_1$-Gruppe oder einen Rest $Y_B$ bedeutet oder $Y_4$ eine $R_2$-Gruppe und $Y_5$ einen Rest $Y_B$ darstellt, wobei $Y_A$ einen in unsubstituiertes oder wie angegeben substituiertes Carbamyl oder, sofern $Y_5$ gegebenenfalls wie angegeben substituiertes Amino $R_1$ darstellt, in Cyano und $Y_B$ einen in gegebenenfalls wie angegeben substituiertes Carbamyl überführbaren Rest bedeutet, oder in einem Salz davon $Y_A$ in Cyano oder gegebenenfalls wie angegeben substituiertes Carbamyl und/oder $Y_B$ in gegebenenfalls wie angegeben substituiertes Carbamyl überführt oder aus einer Verbindung der Formel

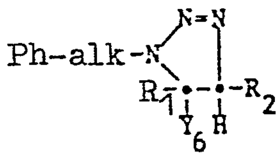 (IX),

worin $Y_6$ einen abspaltbaren Rest bedeutet, $H-Y_6$ abspaltet oder in einer Verbindung der Formel

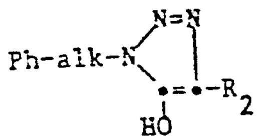 (XII)

die Hydroxygruppe in Niederalkoxy überführt und erforderlichenfalls ein erhaltenes Isomerengemisch in die Komponenten auftrennt und das Isomere der Formel I isoliert und gewünschtenfalls die verfahrensgemäss erhältliche Verbindung in eine andere Verbindung der Formel I umwandelt und/oder ein verfahrensgemäss erhältliche freie Verbindung in ein Salz oder ein verfahrensgemäss erhältliches Salz in die freie Verbindung überführt.

2. Verfahren gemäss Anspruch 1, dadurch gekennzeichnet, dass man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet, beispielsweise, indem man eine Verbindung der Formel

$$Ph - alk - N_3 \quad (II)$$

mit einer Verbindung der Formel

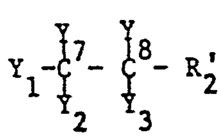 (X),

worin $R'_2$ Cyano oder eine unsubstituierte oder durch Acyl, Niederalkyl oder Niederalkylen bzw. Aza-, Oxa- oder Thianiederalkylen substituierte Carbamylgruppe darstellt, $Y_1$ und $Y_2$ Niederalkoxy oder gegebenenfalls substituiertes Amino $R_1$, $Y_3$ Wasserstoff und $Y_7$ und $Y_8$ eine zusätzliche Bindung, oder $Y_1$, $Y_2$ und $Y_7$ Niederalkoxy und $Y_3$ und $Y_8$ Wasserstoff oder $Y_1$ und $Y_2$ gemeinsam Oxo, $Y_7$ Wasserstoff oder Niederalkyl und $Y_3$ sowie $Y_8$ Wasserstoff bedeuten, oder einem Tautomeren und/oder Salz davon und das Reaktionsprodukt der Formel (IX) ohne Isolierung weiter umsetzt.

3. Verfahren gemäss einem der Ansprüche 1 und 2, dadurch gekennzeichnet, dass man Verbindungen der Formel I, worin Ph durch Niederalkyl, Halogen und/oder Trifluormethyl substituiertes Phenyl bedeutet, alk Niederalkyliden darstellt, $R_1$ Wasserstoff, Niederalkyl, Niederalkoxy, Amino, Acylamino, N-Niederalkyl-, N,N-Diniederalkyl-, N,N-Niederalkylen- bzw. N,N-(Aza-, Oxa- oder Thia)-niederalkylenamino, Carbamyl, N-Acylcarbamyl oder N-Niederalkyl-, N,N-Diniederalkyl-, N,N-Niederalkylen- bzw. N,N-(Aza-, Oxa- oder Thia)-niederalkylencarbamyl bedeutet und $R_2$ für Carbamyl, N-Acylcarbamyl, N-Niederalkyl-, N,N-Diniederalkyl-, N,N-Niederalkylen- bzw. N,N-(Aza-, Oxa- oder Thia)-niederalkylencarbamyl oder, sofern $R_1$ Amino, Acylamino, N-Niederalkyl-, N,N-Diniederalkyl-, N,N-Niederalkylen- bzw. N,N-(Aza-, Oxa- oder Thia)-niederalkylenamino ist, für Cyano bzw., sofern $R_1$ Carbamyl, N-Acylcarbamyl, N-Niederalkyl-, N,N-Diniederalkyl-, N,N-Niederalkylen- bzw. N,N-(Aza-, Oxa- oder Thia)-niederalkylencarbamyl bedeutet, für Wasserstoff oder Niederalkyl steht, wobei Acyl jeweils insbesondere Niederalkanoyl, Niederalkoxyniederalkoxycarbonyl, Diniederalkylaminoniederalkanoyl, Niederalkansulfonyl, Niederalkoxycarbonyl, Ureido, 3-Niederalkyl- bzw. 3,3-Diniederalkylureido oder 3,3-Niederalkylen- bzw. 3,3-(Aza-, Oxa- oder Thia)-niederalkylenureido darstellt und Niederalkyl sowie Niederalkyl in N-Niederalkyl- bzw. N,N-Diniederalkylamino bzw. -carbamyl, Niederalkoxy und Niederalkyliden z.B. bis und mit 7 C-Atome und Niederalkylen bzw. Aza-, Oxa- oder Thianiederalkylen z.B. 4 bis und mit 6 Ringglieder aufweisen, oder ein Salz einer salzbildenden Verbindung der vorstehend definierten Art herstellt.

4. Verfahren gemäss einem der Ansprüche 1 und 2, dadurch gekennzeichnet, dass man Verbindungen der Formel I,

worin Ph 2-Halogenphenyl, 2-Trifluormethylphenyl, 2-Niederalkylphenyl, 2,3- bzw. 2,6-Dihalogenphenyl, 2-Halogen-3-niederalkyl-phenyl bzw. 3-Halogen-2-niederalkylphenyl, 2-Halogen-6-niederalkyl-phenyl, 2,3- bzw. 2,6-Diniederalkylphenyl, 3-Halogen-2-trifluormethyl-phenyl bzw. 2-Halogen-3-trifluormethylphenyl oder 2-Halogen-6-trifluormethylphenyl bedeutet, alk 1,1-Niederalkyliden darstellt, $R_1$ Wasserstoff, Niederalkyl, Niederalkoxy, Amino, Niederalkylamino, Diniederalkylamino, Carbamyl, N-Niederalkylcarbamyl oder N,N-Diniederalkylcarbamyl bedeutet und $R_2$ Carbamyl, N-Niederalkylcarbamyl oder N,N-Diniederalkylcarbamyl bedeutet, wobei Niederalkyl sowie Niederalkyl in N-Niederalkyl bzw. N,N-Diniederalkylamino bzw. -carbamyl, Niederalkoxy und Niederalkyliden z.B. bis und mit 7 C-Atome aufweisen.

5. Verfahren gemäss einem der Ansprüche 1 und 2, dadurch gekennzeichnet, dass man Verbindungen der Formel I, worin Ph durch bis und mit 3 Niederalkylgruppen bzw. Halogenatome, Niederalkyl und Halogen und/oder Trifluormethyl, wobei Niederalkyl mit bis und mit 4 C-Atomen, bzw. Halogen die Atomnummer bis und mit 35 aufweist, substituiertes Phenyl, bedeutet, alk 1,1-Niederalkyliden mit bis und mit 4 C-Atomen darstellt, $R_1$ Amino, Niederalkanoylamino mit bis und mit 7 C-Atomen, Niederalkylamino bzw. Diniederalkylamino mit jeweils bis und mit 4 C-Atomen im Alkylteil darstellt und $R_2$ Carbamyl, N-Niederalkanoylcarbamyl mit bis und mit 7 C-Atomen, N-Niederalkyl-, N,N-Diniederalkyl- oder N,N-Niederalkylencarbamyl mit jeweils bis und mit 4 C-Atomen im Alkyl- bzw. 4 bis und mit 6 Ringgliedern in Alkylenteil oder Cyano bedeutet, herstellt.

6. Verfahren gemäss einem der Ansprüche 1 und 2, dadurch gekennzeichnet, dass man Verbindungen der Formel I,
worin Ph als Niederalkyl- bzw. Halogensubstituenten Niederalkyl mit bis und mit 4 C-Atomen bzw. Halogen der Atomnummer bis und mit 35 aufweisendes 2-Halogenphenyl, 2-Trifluormethylphenyl, 2-Niederalkylphenyl, oder 2,6-Dihalogenphenyl oder 2,3- oder 2,6-Diniederalkylphenyl bedeutet, alk 1,1-Niederalkyliden mit bis und mit 4 C-Atomen darstellt, $R_1$ für Amino, Niederalkylamino bzw. Diniederalkylamino mit jeweils bis und mit 4 C-Atomen im Alkylteil bedeutet und $R_2$ Carbamyl bedeutet, herstellt.

7. Verfahren gemäss einem der Ansprüche 1 und 2, dadurch gekennzeichnet, dass man Verbindungen der Formel I, worin Ph als Niederalkyl- bzw. Halogensubstituenten Niederalkyl mit bis und mit 4 C-Atomen bzw. Halogen der Atomnummer bis und mit 35 aufweisendes 2-Halogenphenyl, 2-Trifluormethylphenyl, 2-Niederalkylphenyl, oder 2,6-Dihalogenphenyl oder 2,3- oder 2,6-Diniederalkylphenyl bedeutet, alk 1,1-Niederalkyliden mit bis und mit 4 C-Atomen darstellt, $R_1$ für Amino, Niederalkylamino bzw. Diniederalkylamino mit jeweils bis und mit 4 C-Atomen im Alkylteil bedeutet und $R_2$ Carbamyl oder Cyano bedeutet, herstellt.

8. Verfahren gemäss einem der Ansprüche 1 und 2, dadurch gekennzeichnet, dass man das 5-Amino-1-(o-chlorbenzyl)-1H-1,2,3-triazol-4-carboxamid herstellt.

9. Verfahren gemäss einem der Ansprüche 1 und 2, dadurch gekennzeichnet, dass man das 5-Amino-1-(2,6-dichlorbenzyl)-1H-1,2,3-triazol-4-carboxamid, das 5-Amino-1-(o-methylbenzyl)-1H-1,2,3-triazol-4-carboxamid, das 5-Amino-1-(o-fluorbenzyl)-1H-1,2,3-triazol-4-carboxamid, das 5-Amino-1-(o-brombenzyl)-1H-1,2,3-triazol-4-carboxamid, das 5-Amino-1-(o-trifluormethylbenzyl)-1H-1,2,3-triazol-4-carboxamid, das 5-Amino-1-(2,3-dimethylbenzyl)-1H-1,2,3-triazol-4-carboxamid, das 5-Amino-1-[1-(o-chlorphenyl)-äthyl]-1H-1,2,3-triazol-4-carboxamid, das 5-Amino-1-[-(o-chlorphenyl)-propyl]-1H-1,2,3-triazol-4-carboxamid, das 5 Amino-1-[1-(o-chlorphenyl)-butyl]-1H-1,2,3-triazol-4-carboxamid, das 5-Amino-1-(o-chlorbenzyl)-1H-1,2,3-triazol-4-carbonsäurenitril, das 1-(o-Chlorbenzyl)-5-dimethylamino-1H-1,2,3-triazol-4-carbonsäure-

nitril, das 1-(o-Chlorbenzyl)-5-dimethylamino-1H-1,2,3-triazol-4-carboxamid, das 1-(o-Chlorbenzyl)-1H-1,2,3-triazol-4,5-dicarboxamid, das 1-(o-Chlorbenzyl)-5-methoxy-1H-1,2,3-triazol-4-carboxamid, das 1-(o-Chlorbenzyl)-1H-1,2,3-triazol-4-carboxamid, das 1-(o-Chlorbenzyl)-5-methyl-1H-1,2,3-triazol-4-carboxamid, das 1-(o-Methylbenzyl)-5-methyl-1H-1,2,3-triazol-4-carboxamid, das 5-Amino-1-(o-chlorbenzyl)-1H-1,2,3-triazol-4-(N-methyl)-carboxamid, das 5-Amino-1-(o-chlorbenzyl)-1H-1,2,3-triazol-4-(N,N-dimethyl)-carboxamid oder das 5-Amino-1-(2,3-dichlorbenzyl)-1H-1,2,3-triazol-4-carboxamid herstellt.

10. Verfahren gemäss einem der Ansprüche 1 und 2, dadurch gekennzeichnet, dass man das 1-(o-Chlorbenzyl)-1H-1,2,3-triazol-4,5-dicarboxamid, das 5-Amino-1-(2,4,6-trimethylbenzyl)-1H-1,2,3-triazol-4-carboxamid, das 5-Amino-1-(5-chlor-2-methylbenzyl)-1H-1,2,3-triazol-4-carboxamid, das 5-Amino-1-(o-chlorbenzyl)-1H-1,2,3-triazol-4-carbonsäurepiperidid, das 5-Amino-1-(m-chlorbenzyl)-1H-1,2,3-triazol-4-carboxamid, das 5-Amino-1-(m-trifluormethylbenzyl)-1H-1,2,3-triazol-4-carboxamid, das 1-(o-Chlorbenzyl)-1H-1,2,3-triazol-4-carbonsäure-piperidid, das 1-(o-Chlorbenzyl)-1H-1,2,3-triazol-4-carbonsäure-N,N-dimethylamid, das 5-Amino-1-(m-methylbenzyl)-1H-1,2,3-triazol-4-carboxamid, das 1-(m-Chlorbenzyl)-1H-1,2,3-triazol-4-carboxamid, das 1-(m-Trifluormethylbenzyl)-1H-1,2,3-triazol-4-carboxamid oder das 5-Amino-1-(o-chlorbenzyl)-1H-1,2,3-triazol-4-carbonsäure-(4-methyl)-piperazid oder ein Säureadditionssalz davon, 5-Acetylamino-1-(o-chlorbenzyl)-1H-1,2,3-triazol-4-carboxamid, 5-Acetylamino-1-(o-chlorbenzyl)-1H-1,2,3-triazol-4-(N-acetyl)carboxamid, 1-(o-Chlorbenzyl)-5-formylamino-1H-1,2,3-triazol-4-carboxamid, 1-(o-Chlorbenzyl)-5-formylamino-1H-1,2,3-triazol-4-(N-formyl)-carboxamid, 5-Aethoxycarbonylamino-1-(o-chlorbenzyl)-1H-1,2,3-triazol-4-carboxamid, 5-Aethoxyacetylamino-1-(o-chlorbenzyl)-1H-1,2,3-triazol-4-carboxamid, 1-(o-Chlorbenzyl)-5-(3,3-dimethylureido)-1H-1,2,3-triazol-4-carboxamid, 1-(o-Chlorbenzyl)-5-(N',N'-dimethylglycylamino)-1H-1,2,3-triazol-4-carboxamid, 1-(o-Chlorbenzyl)-5-(4-methylpiperazinocarbonylamino)-1H-1,2,3-triazol-4-carboxamid herstellt.

11. Verfahren zur Herstellung pharmazeutischer Präparate, dadurch gekennzeichnet, dass man eine gemäss einem der Ansprüche 1 bis 9 erhältliche Verbindung mit üblichen pharmazeutischen Hilfsstoffen vermischt.

12. Verfahren zur Herstellung pharmazeutischer Präparate, dadurch gekennzeichnet, dass man ein Azid der Formel

$$Ph - alk - N_3 \qquad (II)$$

mit einer Verbindung der Formel

$$Y_1 - \underset{Y_2}{\underset{|}{C}} = \underset{Y_3}{\underset{|}{C}} - R_2' \qquad (III),$$

worin $R_2'$ Cyano oder eine unsubstituierte oder durch Acyl, Niederalkyl oder Niederalkylen bzw. Aza-, Oxa- oder Thianiederalkylen substituierte Carbamylgruppe darstellt, $Y_1$ eine wie angegeben disubstituierte Aminogruppe oder, sofern $R_2'$ für eine unsubstituierte oder durch Acyl, Niederalkyl oder Niederalkylen bzw. Aza-, Oxa- oder Thianiederalkylen substituierte Carbamylgruppe steht, Wasserstoff, Niederalkyl, Niederalkoxy oder unsubstituiertes oder wie angegeben substituiertes Carbamyl bedeutet und $Y_2$ und $Y_3$ gemeinsam eine zusätzliche Bindung darstellen, oder worin $R_2'$ Cyano oder eine unsubstituierte oder durch Niederalkyl oder Niederalkylen bzw. Aza-, Oxa- oder Thianiederalkylen substituierte Carbamylgruppe darstellt, $Y_1$ und $Y_2$ gemeinsam für Imino stehen und $Y_3$ Wasserstoff ist, oder einem Tautomeren und/ oder Salz davon umsetzt oder eine Verbindung der Formel

$$Ph - alk - Z \qquad (IV),$$

worin Z reaktionsfähiges verestertes Hydroxy bedeutet, mit einem 1-H-1,2,3-Triazolderivat der Formel

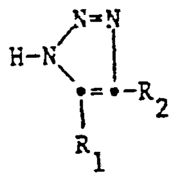

(V)

oder einem Salz davon umsetzt oder in einer Verbindung der Formel

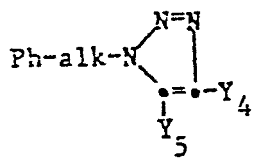

(VI),

worin $Y_4$ einen Rest $Y_A$ und $Y_5$ eine $R_1$-Gruppe oder einen Rest $Y_B$ bedeutet oder $Y_4$ eine $R_2$-Gruppe und $Y_5$ einen Rest $Y_B$ darstellt, wobei $Y_A$ einen in unsubstituiertes oder wie angegeben substituiertes Carbamyl oder, sofern $Y_5$ gegebenenfalls wie angegeben substituiertes Amino $R_1$ darstellt, in Cyano und $Y_B$ einen in gegebenenfalls wie angegeben substituiertes Carbamyl überführbaren Rest bedeutet, oder in einem Salz davon $Y_A$ in Cyano oder gegebenenfalls wie angegeben substituiertes Carbamyl und/oder $Y_B$ in gegebenenfalls wie angegeben substituiertes Carbamyl überführt oder aus einer Verbindung der Formel

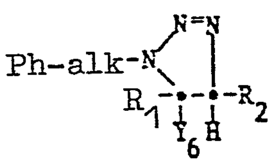

(IX),

worin $Y_6$ einen abspaltbaren Rest bedeutet, H-$Y_6$ abspaltet oder in einer Verbindung der Formel

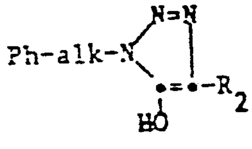

(XII)

die Hydroxygruppe in Niederalkoxy überführt und erforderlichenfalls ein erhaltenes Isomerengemisch in die Komponenten auftrennt und das

Isomere der Formel I isoliert und gewünschtenfalls die verfahrensgemäss erhältliche Verbindung in eine andere Verbindung der Formel I umwandelt und/oder ein verfahrensgemäss erhältliche freie Verbindung in ein Salz oder ein verfahrensgemäss erhältliches Salz in die freie Verbindung überführt, und das Reaktionsprodukt der Formel

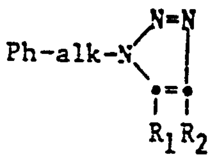

(I),

worin Ph durch Niederalkyl, Halogen und/oder Trifluormethyl substituiertes Phenyl bedeutet, alk Niederalkyliden darstellt, $R_1$ Wasserstoff, Niederalkyl, Niederalkoxy oder eine unsubstituierte oder durch Acyl, Niederalkyl oder Niederalkylen bzw. Aza-, Oxa- oder Thianiederalkylen substituierte Amino- oder Carbamylgruppe bedeutet und $R_2$ für eine unsubstituierte oder durch Acyl, Niederalkyl oder Niederalkylen bzw. Aza-, Oxa- oder Thianiederalkylen substituierte Carbamylgruppe oder, sofern $R_1$ eine unsubstituierte oder durch Acyl, Niederalkyl oder Niederalkylen bzw. Aza-, Oxa- oder Thianiederalkylen substituierte Aminogruppe darstellt, für Cyano bzw., sofern $R_1$ eine unsubstituierte oder durch Acyl, Niederalkyl oder Niederalkylen bzw. Aza-, Oxa- oder Thianiederalkylen substituierte Carbamylgruppe bedeutet, für Wasserstoff oder Niederalkyl steht, mit üblichen pharmazeutischen Hilfsstoffen vermischt.

FO 7.4 KVB/rn*